# EUROPEAN PATENT SPECIFICATION

(11) **EP 1 599 575 B1**
(45) Date of publication and mention of the grant of the patent: **14.09.2011**
(21) Application number: 04713403.6
(22) Date of filing: 20.02.2004
(51) Int. Cl.: A01N 63/00, C12N 5/00

(54) **METHODS OF USING ADIPOSE TISSUE-DERIVED CELLS IN THE TREATMENT OF CARDIOVASCULAR CONDITIONS**
VERFAHREN ZUR VERWENDUNG VON AUS FETTGEWEBE STAMMENDEN ZELLEN BEI DER BEHANDLUNG VON HERZ-KREISLAUF-LEIDEN
METHODES D'UTILISATION DE CELLULES DERIVEES DE TISSUS ADIPEUX DANS LE TRAITEMENT D'ETATS CARDIOVASCULAIRES

(30) Priority: 20.02.2003 US 449279 P; 15.04.2003 US 462911 P
(43) Date of publication of application: 30.11.2005
(73) Proprietor: Cytori Therapeutics, Inc., San Diego, California 92121 (US)
(72) Inventor: FRASER, John, K., Los Angeles, CA 90064 (US); HEDRICK, Marc, H., Encino, CA 91436 (US); ZHU, Min, San Diego, CA 92129 (US); STREM, Brian, M., San Diego, CA 92109 (US); DANIELS, Eric, Santa Clarita, CA 91321 (US); WULUR, Isabella, West Hills, CA 91307 (US)
(74) Representative: Plougmann & Vingtoft A/S
(86) International application number: PCT/US2004/005117
(87) International publication number: WO 2004/074457

(56) References cited:
- WO-A-00/53795
- WO-A-03/053346
- US-A- 4 000 275
- US-A- 4 963 489
- US-A- 5 869 037
- ZUK P A ET AL: "Multilineage cells from human adipose tissue: Implications for cell-based therapies" TISSUE ENGINEERING, LARCHMONT, NY, US, vol. 7, no. 2, April 2001 (2001-04), pages 211-228, XP002198710 ISSN: 1076-3279
- MORIZONO K ET AL: "Multilineage cells from adipose tissue as gene delivery vehicles" HUMAN GENE THERAPY, MARY ANN LIEBERT, NEW YORK ,NY, US, vol. 14, no. 1, 1 January 2003 (2003-01-01), pages 59-66, XP002339340 ISSN: 1043-0342
- ZUK P A ET AL: "Human adipose tissue is a source of multipotent stem cells" MOLECULAR BIOLOGY OF THE CELL, BETHESDA, MD, US, vol. 13, no. 12, 20 December 2002 (2002-12-20), pages 4279-4295, XP002249630 ISSN: 1059-1524
- RANGAPPA S ET AL: "Transformation of adult mesenchymal stem cells isolated from the fatty tissue into cardiomyocytes" THE ANNALS OF THORACIC SURGERY, ELSEVIER, vol. 75, no. 3, 1 March 2003 (2003-03-01), pages 775-779, XP002401034 ISSN: 0003-4975
- GARCIA-OLMO DAMIAN ET AL: "Autologous stem cell transplantation for treatment of rectovaginal fistula in perianal Crohn's disease: A new cell-based therapy" INTERNATIONAL JOURNAL OF COLORECTAL DISEASE, SPRINGER VERLAG, BERLIN, DE, vol. 18, no. 5, September 2003 (2003-09), pages 451-454, XP002359162 ISSN: 0179-1958
- NATHAN SURESH ET AL: "Cell-based therapy in the repair of osteochondral defects: a novel use for adipose tissue." TISSUE ENGINEERING AUG 2003, vol. 9, no. 4, August 2003 (2003-08), pages 733-744, XP002439820 ISSN: 1076-3279
- FULTON J.E. ET AL: 'Fat grafting' DERMATOL CLIN vol. 19, no. 3, July 2001, pages 523 - 530, XP009085713
- ZUK P.A. ET AL: 'Multi lineage cells from human adipose tissue: implications for cell-based therapies' TISSUE ENG vol. 7, no. 2, April 2001, pages 211 - 228, XP002198710
- ZUK P.A. ET AL: 'Human adipose tissue is a source of multipotent stem cells' MOLECULAR BIOLOGY vol. 13, December 2002, pages 4279 - 4295, XP002249630
- GARCIA-OLMO D. ET AL: 'Autologous stem cell transplantation for treatment of rectovaginal fistula in perianal Crohn's disease: a new cell-based therapy.' INT J COLORECTAL DIS vol. 18, no. 5, September 2003, pages 451 - 454, XP002359162
- NATHAN S. ET AL: 'Cell-based therapy in the repair of osteochondral defects: a novel use for adipose tissue' TISSUE ENGINEERING vol. 9, no. 4, August 2003, pages 733 - 744, XP002439820

## Description

### BACKGROUND OF THE INVENTION

### 1. Field of the Invention

This invention generally relates to the use of a composition comprising a concentrated Population of adipose-derived cells (ADC) in the manufacture of a medicament for restoring blood flow in a subject that is evaluated as having an ischemia, wherein said concentrated population of adipose-derived cells (ADC) comprises adipose derived progenitor cells and adipose stem cells obtained by processing adipose tissue from said patient in a system, which is configured to maintain a closed sterile fluid/tissue pathway, said system comprising a collection chamber connected to a mixing/processing chamber.

### 2. Description of Related Art

Cardiovascular diseases and disorders are the leading cause of death and disability in all industrialized nations. In the United States alone, cardiovascular disease accounts for about 40 percent of the mortality rate and affects 58 million Americans (American-Heart-Association, 2002). One of the primary factors that renders cardiovascular disease particularly devastating is the heart's inability to repair itself following damage. Since cardiac muscle cells, i.e., myocardial cells, are unable to divide and repopulate areas of damage, cardiac cell loss as a result of injury or disease is largely irreversible (Abbate et aL, 2002; Remme, 2000).

Of the available forms of therapy, human to human heart transplants have been the most effective in treating severe cardiovascular diseases and disorders. In fact, the one-year and five-year survival rate of the average cardiac transplant recipient is currently over 70 percent. Unfortunately, however, transplantation is a severely limited form of therapy for a number of reasons, namely, the scarcity of suitable donors, the expense of the procedure and the high likelihood of graft rejection and associated problems such as infections, renal dysfunction and immunosuppressant related cancers (American-Heart-Association, 2002).

An alternative to transplant therapy is the use of regenerative medicine to repair and regenerate damaged cardiac muscle cells. Regenerative medicine harnesses, in a clinically targeted manner, the ability of stem cells (i.e., the unspecialized master cells of the body) to renew themselves indefinitely and develop into mature specialized cells. Stem cells are found in embryos during early stages of development, in fetal tissue and in some adult organs and tissue (Pera et al., 2000). Embryonic stem cells (hereinafter referred to as "ESCs") are known to become many if not all of the cell and tissue types of the body. ESCs not only contain all the genetic information of the individual but also contain the nascent capacity to become any of the 200+ cells and tissues of the body. Thus, these cells have tremendous potential for regenerative medicine. For example, ESCs can be grown into specific tissues such as heart, lung or kidney which could then be used to repair damaged and diseased organs (Assady et al., 2001; Jacobson et al., 2001; Odorico et al., 2001). However, ESC derived tissues have clinical limitations. Since ESCs are necessarily derived from another individual, *i.e*., an embryo, there is a risk that the recipient's immune system will reject the new biological material. Although immunosuppressive drugs to prevent such rejection are available, such drugs are also known to block desirable immune responses such as those against bacterial infections and viruses. Moreover, the ethical debate over the source of ESCs, i.e., embryos, is well-chronicled and presents an additional and, perhaps, insurmountable obstacle for the foreseeable future.

Adult stem cells (hereinafter interchangeably referred to as "ASCs") represent an alternative to the use of ESCs. ASCs reside quietly in many non-embryonic tissues, presumably waiting to respond to trauma or other destructive disease processes so that they can heal the injured tissue (Arvidsson et al., 2002; Bonner-Weir and Sharma, 2002; Clarke and Frisen, 2001; Crosby and Strain, 2001; Jiang et al., 2002a). Notably, emerging scientific evidence indicates that each individual carries a pool of ASCs that may share with ESCs the ability to become many if not all types of cells and tissues (Young et al., 2001; Jiang et al., 2002a; Jiang et al., 2002b; Schwartz et al., 2002). Thus, ASCs, like ESCs, have tremendous potential for clinical applications of regenerative medicine.

ASC populations have been shown to be present in one or more of bone marrow, skin, muscle, liver and brain (Jiang et al., 2002b; Alison, 1998; Crosby and Strain, 2001). However, the frequency of ASCs in these tissues is low. For example, mesenchymal stem cell frequency in bone marrow is estimated at between 1 in 100,000 and 1 in 1,000,000 nucleated cells (D'Ippolito et al., 1999; Banfi et al., 2001; Falla et al., 1993). Similarly, extraction of ASCs from skin involves a complicated series of cell culture steps over several weeks (Toma et al., 2001) and clinical application of skeletal muscle-derived ASCs requires a two to three week culture phase (Hagege et al., 2003). Thus, any proposed clinical application of ASCs from such tissues requires increasing cell number, purity, and maturity by processes of cell purification and cell culture.

Although cell culture steps may provide increased cell number, purity, and maturity, they do so at a cost. This cost can include one or more of the following technical difficulties: loss of cell function due to cell aging, loss of potentially useful non-stem cell populations, delays in potential application of cells to patients, increased monetary cost, and increased risk of contamination of cells with environmental microorganisms during culture. Recent studies examining the therapeutic effects of bone-marrow derived ASCs have used essentially whole marrow to circumvent the problems associated with cell culturing (Horwitz et al., 2001; Orlic et al., 2001; Stamm et al., 2003; Strauer et al., 2002). The clinical benefits, however, have been suboptimal, an outcome almost certainly related to the limited ASC dose and purity inherently available in bone marrow.

Recently, adipose tissue has been shown to be a source of ASCs (Zuk et al., 2001; Zuk et al., 2002). WO 00/53795 discloses adipose derived stem cell and lattices, e.g. lipo-derived stem cells substantially free of adipocytes and red blood cells and clonal populations of connective tissue stem cells, The cells can be employed, alone or within biologically-compatible compositions, to generate differentiated tissues and structures, both *in vivo* and *in vitro.* Unlike marrow, skin, muscle, liver and brain, adipose tissue is comparably easy to harvest in relatively large amounts (Commons et al., 2001; Katz et al., 2001b). Furthermore, adipose derived ASCs have been shown to possess the ability to generate multiple tissues *in vitro*, including bone, fat, cartilage, and muscle (Ashjian et al., 2003; Mizuno et al., 2002; Zuk et al., 2001; Zuk et al., 2002). Thus, adipose tissue presents an optimal source for ASCs for use in regenerative medicine. Suitable methods for harvesting adipose derived ASCs, however, are lacking in the art. The existing methods suffer from a number of shortcomings. For example, the existing methods lack the ability to optimally accommodate an aspiration device for removal of adipose tissue. The existing methods also lack partial or full automation from the harvesting of adipose tissue phase through the processing of tissue phases (Katz et al., 2001a). The existing methods further lack volume capacity greater than 100ml of adipose tissue. The existing methods yet further lack a partially or completely closed system from the harvesting of adipose tissue phase through the processing of tissue phases. Finally, the existing methods lack disposability of components to attenuate concomitant risks of cross-contamination of material from one sample to another. In summary, the prior art methods for harvesting ASCs from adipose tissue do not overcome the technical difficulties associated with harvesting ASCs from skin, muscle, liver and brain described above.

Accordingly, given the tremendous therapeutic potential of ASCs, there exists an urgent need in the art for a device, system or method for harvesting ASCs from adipose tissue that produces a population of ASCs with increased yield, consistency and/or purity and does so rapidly and reliably with a diminished or non-existent need for post-extraction manipulation. Ideally, such a device, system or method would yield ASCs in a manner suitable for direct placement into a recipient. Access to such a device, system or method in combination with methods and compositions using adipose derived ASCs for the treatment of cardiovascular diseases and disorders would revolutionize the treatment of such disorders. Given the prevalence of cardiovascular disease and the scarcity of current treatment options, such a treatment is urgently needed.

### SUMMARY OF THE INVENTION

The present invention is based, at least in part, on the discovery that adipose derived adult stem cells can be used to treat cardiovascular conditions, diseases or disorders. The present invention is further based on the discovery of devices, systems and methods for preparing adipose derived adult stem and progenitor cells. The present invention is yet further based on the discovery of methods and compositions of adipose derived adult stem and progenitor cells to treat cardiovascular conditions, diseases or disorders. Accordingly, one aspect of the present invention is directed to the use of a composition comprising a concentrated population of adipose-derived cells (ADC) in the manufacture of a medicament for restoring blood flow in a subject that is evaluated as having an ischemia, wherein said concentrated population of adipose-derived cells (ADC) comprises adipose derived progenitor cells and adipose stem cells obtained by processing adipose tissue from said patient in a system, which is configured to maintain a closed sterile fluid/tissue pathway, said system comprising a collection chamber connected to a mixing/processing chamber.

The adipose tissue processing occurs in a system that maintains a closed, sterile fluid/tissue pathway. This is achieved by use of a pre-assembled, linked set of closed, sterile containers and tubing allowing for transfer of tissue and fluid elements within a closed pathway. This processing set can be linked to a series of processing reagents (e.g., saline, enzymes, etc.) inserted into a device which can control the addition of reagents, temperature, and timing of processing thus relieving operators of the need to manually manage the process. In a preferred approach the entire procedure from tissue extraction through processing and preparation for placement into the recipient would all be performed in the same facility, indeed, even within the same room of the patient undergoing the procedure.

Raw adipose tissue may be processed to substantially remove mature adipocytes and connective tissue thereby obtaining a heterogeneous plurality of adipose tissue-derived cells suitable for placement within the body of a recipient. The cells may be prepared for placement into the recipient in combination with other cells, tissue, tissue fragments, or other stimulators of cell growth and/or differentiation. In a preferred embodiment, the cells, with any of the above mentioned additives, are for placement into person from whom they were obtained in the context of a single operative procedure with the intention of deriving a therapeutic benefit to the recipient.

The cells of the invention may be provided using the method including the steps of: a) providing a tissue removal system; b) removing adipose tissue from a patient using the tissue removal system, the adipose tissue having a concentration of stem cells; c) processing at least a part of the adipose tissue to obtain a concentration of stem cells other than the concentration of stem cells of the adipose tissue before processing for administration of the stem and progenitor cells to a patient without removing the stem and progenitor cells from the tissue removal system before being administered to the patient using several methods known to one of ordinary skill in the art, including but not limited to, intravenous, intracoronary and endomyocardial.

A system used by the invention and disclosed includes a) a tissue collection container including i) a tissue collecting inlet port structured to receive adipose tissue removed from a patient; and ii) a filter disposed within the container and being structured to retain adipose tissue removed from a patient and to pass non-adipose tissue removed from the patient; b) a mixing container coupled to the tissue collection container to receive stem cells obtained from the adipose tissue without removal of the stem cells from the tissue removal system, and including an additive port for the administration of at least one additive to mix with the stem cells contained therein; and c) an outlet structured to permit the cells in the mixing container to be removed from the tissue collection system for administration to a patient

Any feature or combination of features described herein are included within the scope of the present invention provided that the features included in any such combination are not mutually inconsistent as will be apparent from the context, this specification, and the knowledge of one of ordinary skill in the art. Additional advantages and aspects of the present invention are apparent in the following detailed description.

### BRIEF DESCRIPTION OF THE DRAWINGS

Figure 1 depicts a tissue removal system for processing adipose tissue.
Figure 2 depicts a tissue collection container of the tissue removal system of Fig. 1.
Figure 3 is a partial cross-sectional view of the tissue collection container of Fig. 2.
Figure 4 depicts a processing device for automating the operation of a tissue removal system.
Figures 5A and 5B depict the expression of VEGF (5A) and PIGF (5B) protein by cultured adipose derived stem cells.
Figure 6 depicts detection of endothelial progenitor cells within adipose derived stem cell populations.
Figures 7A and 7B depict the *in vitro* development of vascular structures in both normal (7A) and streptozotocin-treated (7B) mice.
Figure 8 depicts the increased average restoration of blood flow in hindlimb ischemia mice treated with adipose derived stem cell compared to a negative control.
Figures 9A and 9B shows that increasing adipose derived stem cell dose improves graft survival and angiogenesis (9A) and depicts the retention of adipose tissue architecture in histologic specimen (9B).
Figure 10 depicts the histological timeline of engraftment of donor derived adipose derived stem cells in the area of infarcted myocardium.
Figure 11 depicts dual positive staining for both beta-galactosidase and myosin heavy chain. Highlighted cells exhibit both blue betagalactosidase staining, demonstrating their origin from donor adipose tissue cells, and brown staining indicating expression of the cardiac muscle protein myosin heavy chain. Cells exhibiting both brown and blue staining (as indicated by arrows) are adipose tissue-derived cells that have taken on the phenotype of cardiac muscle cells.
Figure 12 depicts clusters of donor derived adipose derived stem cells in a region of infarcted myocardium following occlusion/reperfusion injury in the rat.

### DETAILED DESCRIPTION OF THE INVENTION

The present invention provides, for the first time, proven medicaments for treating cardiovascular conditions, diseases and disorders using adipose derived stem and progenitor cells. Specifically, the present invention demonstrates, for the first time, that the adipose derived stem and progenitor cells of the invention (1) express angiogenic and arteriogenic growth factors, including Placenta Growth Factor (PIGF) and Vascular Endothelial Growth Factor (VEGF), (2) contain endothelial progenitor cells (EPC) which have a well-established function in blood vessel formation, (3) develop into blood vessels *in vitro*, (4) support ischemic tissue survival *in vivo*, (5) induce reperfusion following occlusion/reperfusion injury of the hind limb, (6) when injected into animals after heart injury home to the heart, and (7) when injected into an animals after heart injury differentiate into cells expressing markers consistent with their differentiation into cardiac myocytes. Accordingly, the instant disclosure conclusively demonstrates that the inventive adipose derived stem and progenitor cells of the present invention are useful for the treatment of cardiovascular diseases and disorders.

In order that the present invention may be more readily understood, certain terms are first defined. Additional definitions are set forth throughout the detailed description.

As used herein, the term "adipose tissue" refers to a tissue containing multiple cell types including adipocytes and microvascular cells. Adipose tissue includes stem cells and endothelial precursor cells. Accordingly, adipose tissue refers to fat including the connective tissue that stores the fat.

As used herein, the term "unit of adipose tissue" refers to a discrete or measurable amount of adipose tissue. A unit of adipose tissue may be measured by determining the weight and/or volume of the unit. Based on the data identified above, a unit of processed lipoaspirate, as removed from a patient, has a cellular component in which at least 0.1% of the cellular component is stem cells. In reference to the disclosure herein, a unit of adipose tissue may refer to the entire amount of adipose tissue removed from a patient, or an amount that is less than the entire amount of adipose tissue removed from a patient. Thus, a unit of adipose tissue may be combined with another unit of adipose tissue to form a unit of adipose tissue that has a weight or volume that is the sum of the individual units.

As used herein, the term "portion" refers to an amount of a material that is less than a whole. A minor portion refers to an amount that is less than 50%, and a major portion refers to an amount greater than 50%. Thus, a unit of adipose tissue that is less than the entire amount of adipose tissue removed from a patient is a portion of the removed adipose tissue.

As used herein, the term "stem cell" refers to multipotent cells with the potential to differentiate into a variety of other cell types, which perform one or more specific functions and have the ability to self-renew. Some of the stem cells disclosed herein may be pluripotent.

As used herein, the term "progenitor cell" refers to unipotent, bipotent, or multipotent cells with the ability to differentiate into one or more cell types, which perform one or more specific functions and which have limited or no ability to self-renew. Some of the progenitor cells disclosed herein may be pluripotent.

As used herein "stem cell number" or "stem cell frequency" refers to the number of colonies observed in a clonogenic assay in which adipose derived cells (ADC) are plated at low cell density (<10,000 cells/well) and grown in growth medium supporting MSC growth (for example, DMEM/F12 medium supplemented with 10% fetal calf serum, 5% horse serum, and antibiotic/antimycotic agents. Cells are grown for two weeks after which cultures are stained with hematoxylin and colonies of more than 50 cells are counted as CFU-F. Stem cell frequency is calculated as the number of CFU-F observed per 100 nucleated cells plated (for example; 15 colonies counted in a plate initiated with 1,000 nucleated ADC cells gives a stem cell frequency of 1.5%). Stem cell number is calculated as stem cell frequency multiplied by the total number of nucleated ADC cells obtained. A high percentage (∼100%) of CFU-F grown from ADC cells express the cell surface molecule CD105 which is also expressed by marrow-derived stem cells (Barry et al., 1999). CD105 is also expressed by adipose tissue-derived stem cells (Zuk et al., 2002).

As used herein, the term "processed lipoaspirate" refers to adipose tissue that has been processed to separate the active cellular component (e.g., the component containing stem and progenitor cells) from the mature adipocytes and connective tissue. This fraction is referred to herein as "adipose-derived cells" or "ADC." Typically, ADC refers to the pellet of cells obtained by washing and separating the cells from the adipose tissue. The pellet is typically obtained by centrifuging a suspension of cells so that the cells aggregate at the bottom of a centrifuge container.

As used herein, the phrase "cardiovascular condition, disease or disorder" is intended to include all disorders characterized by insufficient, undesired or abnormal cardiac function, *e.g*., ischemic heart disease, hypertensive heart disease and pulmonary hypertensive heart disease, valvular disease, congenital heart disease and any condition which leads to congestive heart failure in a subject, particularly a human subject. Insufficient or abnormal cardiac function can be the result of disease, injury and/or aging. By way of background, a response to myocardial injury follows a well-defined path in which some cells die while others enter a state of hibernation where they are not yet dead but are dysfunctional. This is followed by infiltration of inflammatory cells, deposition of collagen as part of scarring, all of which happen in parallel with in-growth of new blood vessels and a degree of continued cell death. As used herein, the term "ischemia" refers to any localized tissue ischemia due to reduction of the inflow of blood. The term "myocardial ischemia" refers to circulatory disturbances caused by coronary atherosclerosis and/or inadequate oxygen supply to the myocardium. For example, an acute myocardial infarction represents an irreversible ischemic insult to myocardial tissue. This insult results from an occlusive (e.g., thrombotic or embolic) event in the coronary circulation and produces an environment in which the myocardial metabolic demands exceed the supply of oxygen to the myocardial tissue.

As used herein, the term "angiogenesis" refers to the process by which new blood vessels are generated from existing vasculature and tissue (Folkman, 1995). The phrase "repair or remodeling" refers to the reformation of existing vasculature. The alleviation of tissue ischemia is critically dependent upon angiogenesis. The spontaneous growth of new blood vessels provides collateral circulation in and around an ischemic area, improves blood flow, and alleviates the symptoms caused by the ischemia. As used herein, the term "angiogenic factor" or "angiogenic protein" refers to any known protein capable of promoting growth of new blood vessels from existing vasculature ("angiogenesis"). Suitable angiogenic factors for use in the invention include, but are not limited to, Placenta Growth Factor (Luttun et al., 2002), Macrophage Colony Stimulating Factor (Aharinejad et al., 1995), Granulocyte Macrophage Colony Stimulating Factor (Buschmann et al., 2003), Vascular Endothelial Growth Factor (VEGF)-A, VEGF-A, VEGF-B, VEGF-C, VEGF-D, VEGF-E (Mints et al., 2002), neuropilin (Wang et al., 2003), fibroblast growth factor (FGF)-1, FGF-2(bFGF), FGF-3, FGF-4, FGF-5, FGF-6 (Botta et al., 2000), Angiopoietin 1, Angiopoietin 2 (Sundberg et al., 2002), erythropoietin (Ribatti et al., 2003), BMP-2, BMP-4, BMP-7 (Carano and Filvaroff, 2003), TGF-beta (Xiong et al., 2002), IGF-1 (Shigematsu et al., 1999), Osteopontin (Asou et al., 2001), Pleiotropin (Beecken et al., 2000), Activin (Lamouille et al., 2002), Endothelin-1 (Bagnato and Spinella, 2003)and combinations thereof. Angiogenic factors can act independently, or in combination with one another. When in combination, angiogenic factors can also act synergistically, whereby the combined effect of the factors is greater than the sum of the effects of the individual factors taken separately. The term "angiogenic factor" or "angiogenic protein" also encompasses functional analogues of such factors. Functional analogues include, for example, functional portions of the factors. Functional analogues also include anti-idiotypic antibodies which bind to the receptors of the factors and, thus, mimic the activity of the factors in promoting angiogenesis and/or tissue remodeling. Methods for generating such anti-idiotypic antibodies are well known in the art and are described, for example, in WO 97/23510.

Angiogenic factors used in the present invention can be produced or obtained from any suitable source. For example, the factors can be purified from their native sources, or produced synthetically or by recombinant expression. The factors can be administered to patients as a protein composition. Alternatively, the factors can be administered in the form of an expression plasmid encoding the factors. The construction of suitable expression plasmids is well known in the art. Suitable vectors for constructing expression plasmids include, for example, adenoviral vectors, retroviral vectors, adeno-associated viral vectors, RNA vectors, liposomes, cationic lipids, lentiviral vectors and transposons.

As used herein, the term "arteriogenesis" refers to the process of enhancing growth of collateral arteries and/or other arteries from pre-existing arteriolar connections (Carmeliet, 2000; Scholz et al., 2001; Scholz et al., 2002). More particularly, arteriogenesis is the *in situ* recruitment and expansion of arteries by proliferation of endothelial and smooth muscle cells from pre-existing arteriolar connections supplying blood to ischemic tissue, tumor or site of inflammation. These vessels largely grow outside the affected tissue and are important for the delivery of nutrients to the ischemic territory, the tumor or the site of inflammation. Arteriogenesis is part of the normal response to myocardial ischemia (Mills et al., 2000; Monteiro et al., 2003). In addition, the common surgical technique of a coronary artery bypass graft (CABG) is, in effect, no more than creation of an artificial collateral vessel (Sergeant et al., 1997). Thus, processes which enhance arteriogenesis following an infarct will improve blood flow to ischemic tissue resulting in decreased cell death and decreased infarct size. These improvements will result in improved cardiac function and therapeutic benefit.

As used herein, the term "treating" includes reducing or alleviating at least one adverse effect or symptom of a cardiovascular condition, disease or disorder, i.e., any disorder characterized by insufficient or undesired cardiac function. Adverse effects or symptoms of cardiac disorders are well-known in the art and include, but are not limited to, dyspnea, chest pain, palpitations, dizziness, syncope, edema, edema, cyanosis, pallor, fatigue and death.

As used herein, the terms "administering," "introducing" and "transplanting" are used interchangeably herein and refer to the placement of the ADC of the invention into a subject by a method or route which results in at least partial localization of the ADC at a desired site. The ADC can be administered by any appropriate route which results in delivery to a desired location in the subject where at least a portion of the cells or components of the cells remain viable. The period of viability of the cells after administration to a subject can be as short as a few hours, *e.g*., twenty-four hours, to a few days, to as long as several years.

As used herein, the term "subject" includes warm-blooded animals, preferably mammals, including humans. In a preferred embodiment, the subject is a primate. In an even more preferred embodiment, the subject is a human.

Reference will now be made in detail to the presently preferred embodiments of the invention, examples of which are illustrated in the accompanying Figures. Wherever possible, the same or similar reference numbers are used in the drawings and the description to refer to the same or like parts. It should be noted that the drawings are in simplified form and are not to precise scale. In reference to the disclosure herein, for purposes of convenience and clarity only, directional terms, such as, top, bottom, left, right, up, down, over, above, below, beneath, rear, and front, are used with respect to the accompanying drawings. Such directional terms should not be construed to limit the scope of the invention in any manner.

Although the disclosure herein refers to certain illustrated embodiments, it is to be understood that these embodiments are presented by way of example and not by way of limitation. The intent of the following detailed description, although discussing exemplary embodiments, is to be construed to cover all modifications, alternatives, and equivalents of the embodiments as may fall within the spirit and scope of the invention as defined by the appended claims. The present invention may be practiced in conjunction with various cell or tissue separation techniques that are conventionally used in the art, and only so much of the commonly practiced process steps are included herein as are necessary to provide an understanding of the present invention.

Accordingly, one aspect of the present invention is directed to the use of a composition comprising a concentrated population of adipose-derived cells (ADC) in the manufacture of a medicament for restoring blood flow in a subject that is evaluated as having an ischemia, wherein said concentrated population of adipose-derived cells (ADC) comprises adipose derived progenitor cells and adipose stem cells obtained by processing adipose tissue from said patient in a system, which is configured to maintain a closed sterile fluid/tissue pathway, said system comprising a collection chamber connected to a mixing/processing chamber. The cells of the population may be administered to a patient suffering from a cardiovascular disease or disorder without other adipocytes or connective tissue.

In particular, the present invention is directed to use of a composition comprising a concentrated population of adipose tissue-derived cells for the preparation of a medicament for minimizing damage and promoting myocardial repair and regeneration during this process. These include, among others: the ability to synthesize and secrete growth factors stimulating new blood vessel formation; the ability to synthesize and secrete growth factors stimulating cell survival and proliferation; the ability to proliferate and differentiate into cells directly participating in new blood vessel formation; the ability to engraft damaged myocardium and inhibit scar formation (collagen deposition and cross-linking); the ability to proliferate and differentiate into muscle cells capable of contributing to myocardial contractility; and the ability to proliferate and differentiate into myocardial cells.

### I. Methods disclosed herein

### 1. Methods of Obtaining Processed Lipoaspirate (ADC)

It has been discovered that adipose tissue is an especially rich source of stem and progenitor cells. This finding may be due, at least in part, to the ease of removal of the major non-stem cell component of adipose tissue, the adipocyte. Thus, in both human and animal studies, processed lipoaspirate (ADC) contains stem cells at a frequency of at least 0.1%, and more typically greater than 0.5%. In certain embodiments of the invention, ADC has been obtained which contains between about 2-12% stem cells. In even further embodiments, the ADC is processed to obtain a population of cells where the stem cells constitute up to 100% of the cells in the population. The purity/frequency of stem cells obtained in accordance with the invention herein disclosed is substantially greater than the published frequency of 1 in 100,000 (0.001%) in marrow (D'Ippolito et al., 1999; Banfi et al., 2001; Falla et al., 1993; Muschler et al., 2001). Furthermore, collection of adipose tissue is associated with lower morbidity than collection of a similar volume of marrow (Nishimori et al., 2002). In addition, adipose tissue contains endothelial precursor cells, which are capable of providing therapy to patients (see (Asahara et al., 1999; Kaushal et al., 2001; Kawamoto et al., 2003; Kawamoto et al., 2001)).

The cells that are for administration to a patient are obtained from adipose tissue. Adipose tissue can be obtained by any method known to a person of ordinary skill in the art. For example, adipose tissue may be removed from a patient by suction-assisted lipoplasty, ultrasound-assisted lipoplasty, and excisional lipectomy. In addition, the procedures may include a combination of such procedures, such as a combination of excisional lipectomy and suction-assisted lipoplasty. As the tissue or some fraction thereof is intended for re-implantation into a patient the adipose tissue should be collected in a manner that preserves the viability of the cellular component and that minimizes the likelihood of contamination of the tissue with potentially infectious organisms, such as bacteria and/or viruses. Thus, the tissue extraction should be performed in a sterile or aseptic manner to minimize contamination. Suction assisted lipoplasty may be desirable to remove the adipose tissue from a patient as it provides a minimally invasive method of collecting tissue with minimal potential for stem cell damage that may be associated with other techniques, such as ultrasound assisted lipoplasty.

For suction-assisted lipoplastic procedures, adipose tissue is collected by insertion of a cannula into or near an adipose tissue depot present in the patient followed by aspiration of the adipose into a suction device. A small cannula may be coupled to a syringe, and the adipose tissue may be aspirated using manual force (Asken, 1990). Using a syringe or other similar device may be desirable to harvest relatively moderate amounts of adipose tissue (e.g., from 0.1 ml to several hundred milliliters of adipose tissue). Procedures employing these relatively small devices have the advantage that the procedures can be performed with only local anesthesia, as opposed to general anesthesia. Larger volumes of adipose tissue above this range (e.g., greater than several hundred milliliters) may require general anesthesia at the discretion of the donor and the person performing the collection procedure. When larger volumes of adipose tissue are desired to be removed, relatively larger cannulas and automated suction devices may be employed in the procedure (Commons et al., 2001).

Excisional lipectomy procedures include, and are not limited to, procedures in which adipose tissue-containing tissues (e.g., skin) is removed as an incidental part of the procedure; that is, where the primary purpose of the surgery is the removal of tissue (e.g., skin in bariatric or cosmetic surgery) and in which adipose tissue is removed along with the tissue of primary interest.

The adipose tissue that obtained from a patient is collected into a device for further processing. As discussed herein, the device is designed for and dedicated to the purpose of collecting tissue for manufacture of a processed adipose tissue cell population, which includes stem cells and/or endothelial precursor cells. The device may be any conventional device that is typically used for tissue collection by physicians performing the extraction procedure.

The amount of tissue collected will be dependent on a number of variables including, but not limited to, the body mass index of the donor, the availability of accessible adipose tissue harvest sites, concomitant and pre-existing medications and conditions (such as anticoagulant therapy), and the clinical purpose for which the tissue is being collected. Experience with transplant of hematopoietic stem cells (bone marrow or umbilical cord blood-derived stem cells used to regenerate the recipient's blood cell-forming capacity) shows that engraftment is cell dose-dependent with threshold effects (Smith and Sweetenham, 1995; Barker et al., 2001). Thus, it is likely that the general principle that "more is better" will be applied within the limits set by other variables and that where feasible the harvest will collect as much tissue as possible.

It has been discovered that the stem cell percentage of 100 ml of adipose tissue extracted from a lean individual is greater than that extracted from an obese donor (Table 1). This reflects a dilutive effect of the increased fat content in the obese individual. Therefore, it may be desirable to obtain larger amounts of tissue from overweight donors compared to the amounts that would be withdrawn from leaner patients. This observation also indicates that the utility of this invention is not limited to individuals with large amounts of adipose tissue.

**Table 1: Effect of Body Mass Index on Tissue and Cell Yield**

| Body Mass Index Status | Amount of Tissue Obtained (g) | Total Cell Yield (x10⁷) |
|---|---|---|
| Normal | 641±142 | 2.1±0.4 |
| Obese | 1,225±173 | 2.4±0.5 |
| p value | 0.03 | 0.6 |

The concentrated stem cells may be for administration a composition comprising adipose-derived stem cells and/or endothelial precursor cells substantially free from mature adipocytes and connective tissue. In certain embodiments, the composition has a cellular component in which at least 0.1% of the cells are stem cells. In other embodiments, the composition has a cellular component in which the stem cells comprise between about 2% and 12% of the cellular component. Higher concentrations of stem cells, such as up to 100%, are also included in different compositions. The composition may include additional components, such as cell differentiation factors, growth promoters, immunosuppressive agents, or medical devices, as discussed herein. To obtain certain compositions in which the composition primarily contains one type of cell (e.g., adipose-derived stem cells or adipose-derived endothelial precursor cells), any suitable method for separating the different cell types may be employed, such as the use of cell-specific antibodies that recognize and bind antigens present on either stem cells or endothelial precursor cells.

For most applications preparation of the active cell population will require depletion of the mature fat-laden adipocyte component of adipose tissue. This is typically achieved by a series of washing and disaggregation steps in which the tissue is first rinsed to reduce the presence of free lipids (released from ruptured adipocytes) and peripheral blood elements (released from blood vessels severed during tissue harvest), and then disaggregated to free intact adipocytes and other cell populations from the connective tissue matrix. In certain embodiments, the entire adipocyte component, or non-stem cell component, is separated from the stem cell component of the adipose tissue. In other embodiments, only a portion or portions of the adipocyte component is separated from the stem cells. Thus, in certain embodiments, the stem cells can be administered with endothelial precursor cells.

Rinsing is an optional, but preferred, step in which the tissue is mixed with solutions to wash off free lipid and single cell components, such as those components in blood, leaving behind intact adipose tissue fragments. In one embodiment, the adipose tissue that is removed from the patient is mixed with isotonic saline or other physiologic solution(s) (e.g., Plasmalyte®, of Baxter Inc. or Normoso ® of Abbott Labs). Intact adipose tissue fragments can be separated from the free lipid and cells by any means known to persons or ordinary skill in the art including, but not limited to, filtration, decantation, sedimentation, or centrifugation. In the illustrated embodiment of the invention, the adipose tissue is separated from non-adipose tissue by employing a filter disposed within a tissue collection container, as discussed herein. In other embodiments, the adipose tissue is separated from non-adipose tissue using a tissue collection container that utilizes decantation, sedimentation, and/or centrifugation techniques to separate the materials.

The intact tissue fragments are then disaggregated using any conventional techniques or methods, including mechanical force (mincing or shear forces), enzymatic digestion with single or combinatorial proteolytic enzymes, such as collagenase, trypsin, lipase, liberase H1, or members of the Blendzyme family as disclosed in U.S. Pat. No. 5,952,215, and pepsin, or a combination of mechanical and enzymatic methods. For example, the cellular component of the intact tissue fragments may be disaggregated by methods using collagenase-mediated dissociation of adipose tissue, similar to the methods for collecting microvascular endothelial cells in adipose tissue, as disclosed in U.S. Patent No. 5,372,945. Additional methods using collagenase that may be used are disclosed in U.S. Patent No. 5,830,714 and 5,952,215, and by Williams et al., 1995 (Williams et al., 1995). Similarly, a neutral protease may be used instead of collagenase, as disclosed in (Twentyman and Yuhas, 1980) Furthermore, methods may employ a combination of enzymes, such as a combination of collagenase and trypsin, as disclosed in (Russell et al., 1976); or a combination of an enzyme, such as trypsin, and mechanical dissociation, as disclosed in (Engelholm et al., 1985).

The active cell population (processed lipoaspirate) may then be obtained from the disaggregated tissue fragments by reducing the presence of mature adipocytes. A suspension of the processed lipoaspirate and the liquid in which the adipose tissue was disaggregated is then passed to another container, such as a cell collection container. The suspension may flow through one or more conduits to the cell collection container by using a pump, such as a peristaltic pump, that withdraws the suspension from the tissue collection container and urges it to the cell collection container. Other embodiments may employ the use of gravity or a vacuum while maintaining a closed system. Separation of the cells in the suspension may be achieved by buoyant density sedimentation, centrifugation, elutriation, filtration, differential adherence to and elution from solid phase moieties, antibody-mediated selection, differences in electrical charge; immunomagnetic beads, fluorescence activated cell sorting (FACS), or other means. Examples of these various techniques and devices for performing the techniques may be found in (Hemstreet et al., 1980; Schweitzer et al., 1995; Gryn et al., 2002; Prince et al., 2002; Watts et al., 2002; Mainwaring and Rowley, 1985; Greenberg and Hammer, 2001) and U.S. Pat. Nos. 6,277,060; 6,221,315; 6,043,066; 6,451,207; 5,641,622; and 6,251,295.

In the illustrated system, the cells in the suspension are separated from the acellular component of the suspension using a spinning membrane filter. In other embodiments, the cells in the suspension are separated from the acellular component using a centrifuge. In one such exemplary system, the cell collection container may be a flexible bag that is structured to be placed in a centrifuge (e.g., manually or by robotics). In other embodiments, a flexible bag is not used. After centrifugation, the cellular component forms a pellet, which may then be resuspended with a buffered solution so that the cells can be passed through one or more conduits to a mixing container, as discussed herein. The resuspension fluids may be provided by any suitable means. For example, a buffer may be injected into a port on the cell collection container, or the cell collection container may include a reserve of buffer that can be mixed with the pellet of cells by rupturing the reserve. When a spinning membrane filter is used, resuspension is optional since the cells remain in a volume of liquid after the separation procedure.

Although the adipose tissue may be fully disaggregated to separate the active cells from the mature adipocytes and connective tissue, it may in one of the implementations be only partially disaggregated. For example, partial disaggregation may be performed with one or more enzymes, which are removed from the at least a part of the adipose tissue early, relative to an amount of time that the enzyme would otherwise be left thereon to fully disaggregate the tissue. Such a process may require less processing time.

In one particular embodiment, the tissue is washed with sterile buffered isotonic saline and incubated with collagenase at a collagenase concentration, temperature, and time sufficient to provide adequate disaggregation. In a preferred embodiment, the collagenase enzyme used will be approved for human use by the relevant authority (e.g., the U.S. Food and Drug Administration). Suitable collagenase preparations include recombinant and non-recombinant collagenase. Non-recombinant collagenase may be obtained from F. Hoffmann-La Roche Ltd, Indianapolis, IN and/or Advance Biofactures Corp., Lynbrook, NY. Recombinant collagenase may also be obtained as disclosed in U.S. Patent No. 6,475,764.

In one embodiment, solutions contain collagenase at concentrations from about 10 µg/ml to about 50 µglml and are incubated at from about 30°C to about 38°C for from about 20 minutes to about 60 minutes. These parameters will vary according to the source of the collagenase enzyme, optimized by empirical studies, in order to validate that the system is effective at extracting the desired cell populations in an appropriate time frame. A particular preferred concentration, time and temperature is 20 µg/ml collagenase (mixed with the neutral protease disease; Blendzyme 1, Roche) incubated for 45 minutes, at about 37° C. An alternative preferred embodiment applies 0:5 units/mL collagenase (mixed with the neutral protease thermolysin; Blendzyme 3). In a particularly preferred embodiment the collagenase enzyme used is material approved for human use by the relevant authority (e.g., the U.S. Food and Drug Administration). The collagenase used should be free of micro-organisms and contaminants, such as endotoxin.

Following disaggregation the active cell population may be washed/rinsed to remove additives and/or by-products of the disaggregation process (e.g., collagenase and newly-released free lipid). The active cell population could then be concentrated by centrifugation or other methods known to persons of ordinary skill in the art, as discussed above. These post-processing wash/concentration steps may be applied separately or simultaneously.

In one embodiment, the cells are concentrated and the collagenase removed by passing the cell population through a continuous flow spinning membrane system or the like, such as, for example, the system disclosed in U.S. Patent Numbers 5,034,135; and 5,234,608.

In addition to the foregoing, there are many post-wash methods that may be applied for further purifying the active cell population. These include both positive selection (selecting the target cells), negative selection (selective removal of unwanted cells), or combinations thereof.

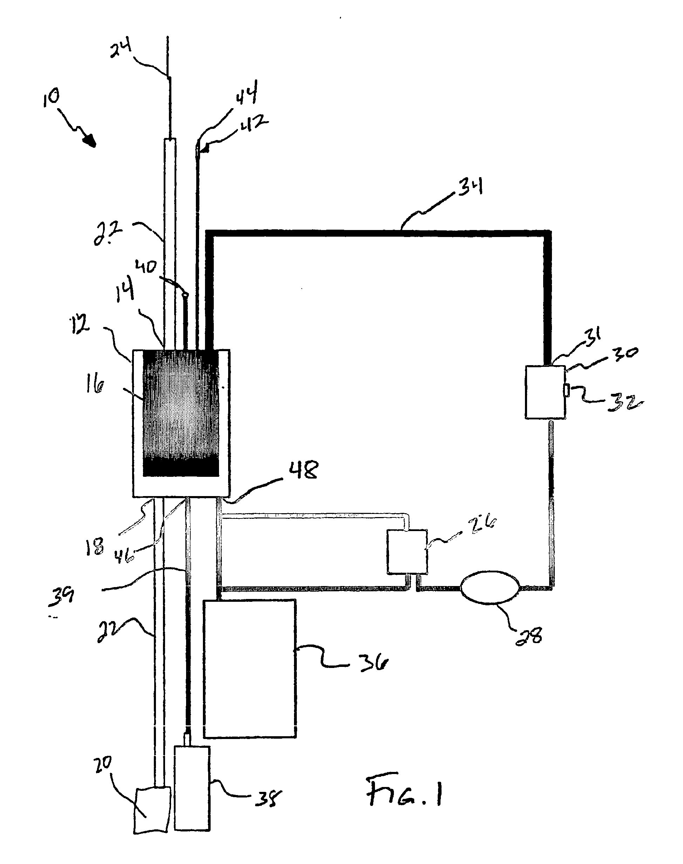

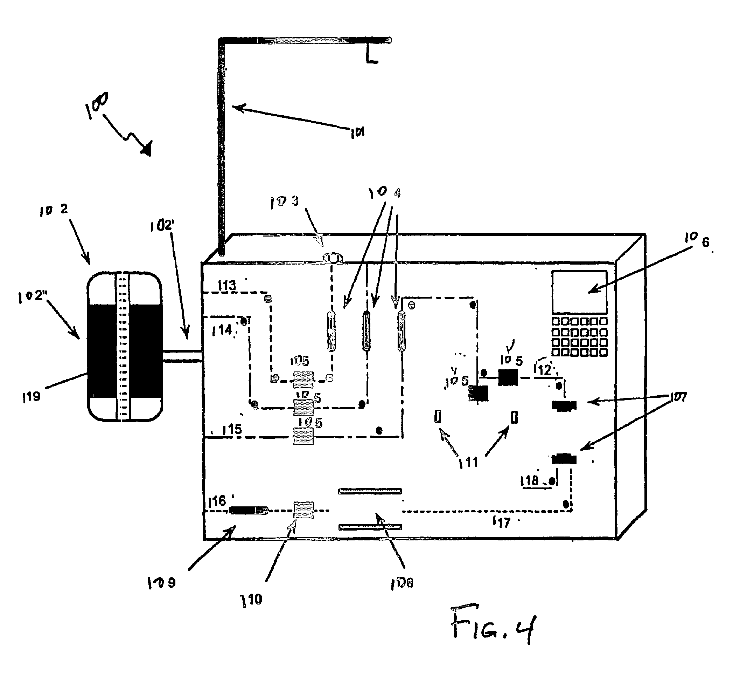

In one embodiment, a solid phase material with adhesive properties selected to allow for differential adherence and/or elution of a subpopulation of cells within the processed lipoaspirate is inserted into the system after the cell washing step. This general approach has been performed in clinical blood transfusion in which filters differentially capturing leukocytes are used to deplete transfused red cells of contaminating white blood cell (Soli et al., 2001). Filters of this type are distributed by Pall Bedical (Leukogard RS and Purecell RCQ) and Asahi (RS2000). Differential adherence has also been applied to positive selection of monocytes (Berdel et al., 1982) and epidermal stem cells (Bickenbach and Dunnwald, 2000). In this embodiment the processed lipoaspirate would be passed through a filter material under flow and buffer conditions pre-determined to promote differential adherence of target cells and unwanted cell populations. For positive selection the filter material and conditions would allow preferential adherence of target cells while unwanted material would pass freely through the filter and be washed away with excess buffer. Target cells would be eluted from the filter by changing the conditions such as flow rate, pH, ionic strength, and/or presence of cations necessary for adhesion. The filter material could be in the form of a three-dimensional mesh, packed cassette of small particles, hollow-fibers or other mechanism with high surface area. In a preferred embodiment, this filter device would be an integral part of the disposable set shown in Figure 1 and would be inserted into the device shown in Figure 4. Both the set and device would have to be modified slightly from those examples shown in the specified figures; Figure 1 to include the filter and housing and Figure 4 to allow for insertion of the filter housing and tubing (including valves) necessary for maintenance of a closed, sterile fluid pathway. Alternatively the mixing chamber (Component 108 of Figure 4; component 30 of Figure 1) could be replaced by the device fittings and filter/housing respectively.

An alternate embodiment of this differential adherence approach would include use of antibodies and/or combinations of antibodies recognizing surface molecules differentially expressed on target and unwanted cells. Selection on the basis of expression of specific cell surface markers (or combinations thereof) is another commonly applied technique in which antibodies are attached (directly or indirectly) to a solid phase support structure (Geiselhart et al., 1996; Formanek et al., 1998; Graepler et al., 1998; Kobari et al., 2001; Mohr et al., 2001). This approach has obvious applications in both positive and negative selection in which, for example, residual white blood cells might be removed by use of the CD45 antibody). Similarly, Reyes et al have applied a complex blend of antibodies in the selection of a multipotential adult progenitor cell from human bone marrow (Reyes et al., 2001). For example, an antibody such as AP2 (Joyner et al., 1999) which specifically binds to adipocytic cells could be employed to preferentially deplete residual adipocytic cells (including immature adipocytes and adipoblasts). Positive selection could be applied by use of antibodies specific for the target cell population(s). For example, Quirici *et al.* have used antibodies to the Nerve Growth Factor Receptor to enrich bone marrow-derived mesenchymal stem cells (Quirici et al., 2002).

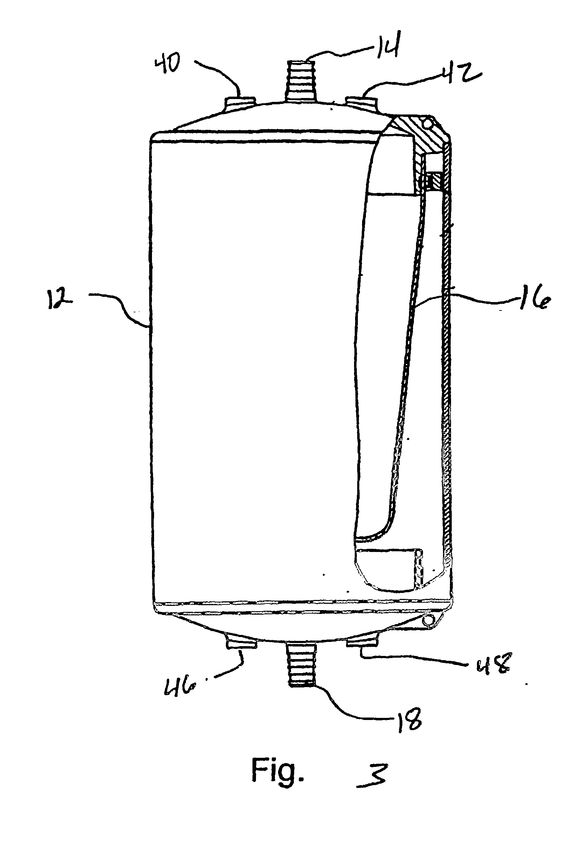

In one embodiment of an antibody-based approach, an antibody (for example AP2) or a cocktail of antibodies (for example AP2, CD3, CD19, CD11b) would be added to the processed lipoaspirate. Many other antibodies and combinations of antibodies will be recognized by one skilled in the art and these examples are provided by way of example only. After incubation, under conditions pre-determined to allow for optimal binding of these antibodies to their cognate antigens, the cells would be washed by passing through the spinning membrane filter or other embodiment of the cell washing chamber to remove unbound, excess antibody. The cells would then be passed over a solid phase structure similar to that described in the embodiment above but in which the solid phase has attached a secondary antibody capable of high affinity attachment to the primary antibodies now bound to the cell surface. Target cells, for example the adipose tissue-derived stem cell, would pass freely through this filter by virtue of the absence of expression of cell surface antigens recognized by the selected antibody (antibody cocktail) thereby creating a negative selection system. In this system the disposable set (Figure 3) and device (Figure 4) would be subject to minor modifications very similar to those described in the above embodiment.

An antibody-mediated positive selection embodiment could be achieved in very similar fashion by including a third additive that facilitates detachment of the cells from the solid phase support. In this embodiment, the enzyme papain or chymopapain could be added to cleave the antibody molecules and release cells from the solid phase support (Civin et al., 1990). Another alternative would be the use of specific peptides that would compete with the cell surface antigen for binding to the antibodies, as described by Tseng-Law et al., US Patent No. 6,017,719.

In another embodiment the cell pellet could be resuspended, layered over (or under) a fluid material formed into a continuous or discontinuous density gradient and placed in a centrifuge for separation of cell populations on the basis of cell density. Examples of media suitable for formation of such gradients include Percoll and Ficoll-Paque (Qian et al., 1998) (Smits et al., 2000) or Ficoll-Paque (Lehner and Holter, 2002; Van, V et al., 2001). This embodiment would be capable of separating out certain residual blood cell populations and immature adipocytes (pre-adipocytes) from the cell population.

In a similar embodiment continuous flow approaches such as apheresis (Smith, 1997), and elutriation (with or without counter-current) (Lasch et al., 2000) (Ito and Shinomiya, 2001) may also be employed. Such mechanisms have been used to fractionate blood cells, including separation of red blood cells on the basis of age (Lasch et al., 2000) and application of this general approach to further purification of cells of interest from processed lipoaspirate will be readily apparent to one skilled in the art. This embodiment may require modification of the device in Figure 4 and the disposable set (Figure 3) such that the device would be integrated with a second device providing the apheresis or elutriation capability.

Adherence to plastic followed by a short period of cell expansion has also been applied in bone marrow-derived adult stem cell populations (Jaiswal et al., 2000). This approach uses culture conditions to preferentially expand one population while other populations are either maintained (and thereby reduced by dilution with the growing selected cells) or lost due to absence of required growth conditions. Sekiya et al have described conditions which might be employed in this regard for bone marrow-derived stem cells (Sekiya et al., 2002). This approach (with or without differential adherence to the tissue culture plastic) could be applied to a further embodiment of this invention. In this approach the cells are removed from the device shown in Figure 4 and placed into a second device providing the cell culture component. This could be in the form of a conventional laboratory tissue culture incubator or a Bioreactor-style device such as that described by Tsao et al., US Patent No. 6,001,642, or by Armstrong et al., US Patent No. 6,238,908. In an alternative embodiment, the mixing component (component 108 of the device shown in Figure 4; component 30 in Figure 3) could be replaced by a Bioreactor component allowing for short-term adherence and/or cell culture of the processed lipoaspirate. This alternate approach would permit integration of the Bioreactor component to the device and remove the need for removing the cells from this device and placement within another.

### i. Illustrated Method for Obtaining Processed Lipoaspirate

An example of a tissue removal system for removing adipose tissue from a patient is illustrated in Fig. 1. In a broad implementation of the system used by the invention, tissue removal system 10 includes a tissue collecting container 12 and a mixing container 30 coupled to the tissue collecting container 12. The coupling between mixing container 30 and tissue collecting container 12 preferably defines a closed system in which the tissue that is directed from tissue collecting container 12 to mixing container 30 is not exposed to the external environment. System 10 also includes an outlet 32 that is structured to permit concentrated stem cells to be removed from tissue collection system 10 to be administered to a patient. The tissue collection container 12 includes a tissue collecting inlet port 14 and a filter 16. Filter 16 is disposed within the container, and is structured to retain adipose tissue and to pass non-adipose tissue as, for example, the tissues are removed from the patient. More specifically, filter 16 allows passage of free lipid, blood, and saline, while retaining fragments of adipose tissue during, or in another embodiment after, the initial harvesting of the adipose tissue. In that regard, filter 16 includes a plurality of pores, of either the same or different sizes, but ranging in size from about 20 µm to 5 mm. In a preferred system, the filter is a medical grade polyester mesh of around 200 µm thickness with a pore size of around 265 µm and around 47% open area. This material holds the tissue during rinsing but allows cells to pass out through the mesh following tissue disaggregation. Thus, when the tissues are aspirated from the patient, the non-adipose tissue may be separated from the adipose tissue. Mixing container 30 includes an additive port 31 that is structured to allow a user to administer an additive to the mixing container 30 to mix with stem cells contained in the mixing container 30. In a preferred system, the dimensions of the tissue collection container 12 should be such as to allow retention of approximately 1 liter of tissue fragments within the filter. In other systems, the tissue collection container 12 may be sized to hold a greater or smaller volume of tissue fragments; for example, the tissue collection container may be sized to store at least 100 mL of adipose tissue fragments, and up to about 2 L of adipose tissue fragments.

Referring to additional features present in system 10 of Fig. 1, tissue inlet port 14 is coupled to cannula 24 by way of tubing 22 to define a tissue removal line. In the illustrated embodiment, cannula 24 is an integrated, single-use liposuction cannula, and the tubing is a flexible tubing. The cannula is dimensioned to be inserted into a patient to remove adipose tissue from the patient. The tubing 22 used in the system should be capable of withstanding negative pressure associated with suction assisted lipoplasty to reduce the likelihood of collapsing. Tissue collection container 12 also includes an aspiration port 18 disposed on the opposite side of filter 16 from tissue inlet port 14. Aspiration port 18 is structured to be coupled to a suction device 20, which may be manually or automatically operated. Suction device 20 may be a syringe or may be an electric vacuum, among other things. Suction device 20 should be capable of providing a sufficient negative pressure to container 12 and cannula 24 to aspirate tissue from a patient. As illustrated, suction device 20 is coupled to aspiration port 18 by way of tubing 22.

Tissue removal system 10 is illustrated as also including a cell collection container 26 positioned between tissue collection container 12 and mixing container 30. Cell collection container 26 is positioned within system 10 so that cells, such as stem cells, pass from tissue collection container 12 to the cell collection container 26 before being passed to mixing container 30. In the illustrated system, cell collection container 26 is coupled to tissue collection container 12 by way of cell collecting port 48. In one version of system 10, cell collection container 26 includes a cell concentrator (not shown) that facilitates separation of the cells in a suspension. An example of a cell concentrator is a centrifuge device that may separate cells from other materials based on, for example, the size or density of the cells. Another example is a spinning membrane filter, as discussed above. System 10 is also illustrated as including a filter 28 structured to pass the cells from cell collection container 26 to mixing container 30, and to prevent passage of material that is, for example, larger than, the cells. Cell collection container 26 also includes an outlet to waste container 36. The direction of flow of the material contained in cell collection container 26 is determined by the positioning of one or more valves which can control whether the material flows to waste container 36 or mixing container 30.

In the illustrated system, cell filter 28 comprises a plurality of pores having a diameter, or length less than 200 µm. In certain systems, the pores may have diameters that are smaller than 200 µm. In other systems, the pores have diameters between 20 and 200 µm. Cell filter 28 may be spaced apart from cell collection container 26 or may be contained within cell collection container 26. Cell filter 28 may also be integrally formed in cell collection container 26. Additional systems of system 10 do not include filter 28. Cell collection container may be fabricated from any suitable material. For example, cell collection container 26 may be a plastic bag, such as those conventionally used in processing blood in blood banks; or in other systems it may be structurally rigid. In certain systems, cell collection container 26 may include a component preparation chamber and a cell washing/separation chamber.

In certain systems, the component preparation chamber includes one or more ports for addition of agents that can enhance the process of separating stem cells for administering to a patient, such as growth factors or buffers for resuspending the cells, as discussed above. In these systems, component preparation chamber preferably includes a mixing device to mix or agitate the cells and additives in the container. Component preparation chamber also includes one or more ports for removing the cells collected therein. One port may be provided to pass the cells toward mixing container 30. Other ports may be provided to direct cells, or a portion of the cells, to other targets, such as implant materials, including bone fragments, or to cell culturing or purification devices. In one system, the cell washing/separation chamber includes a spinning membrane filter component, which may be used as the cell concentrator in addition to or, preferably, as an alternative to a centrifuge device.

System 10 is also illustrated as including a tissue retrieval line 34 which is positioned to provide a conduit from tissue collection container 12 to mixing container 30. Thus, tissue retrieval line 34 passes or directs tissue contained within tissue collection container 12 to mixing container 30 where the tissue can be mixed with cells obtained from cell collection container 26. In the illustrated system, tissue retrieval line 34 extends into tissue container 12 to remove adipose tissue that is contained in filter 16. Tissue is passed or directed through tissue retrieval line 34 using one or more pumps or suction devices to pass adipose tissue that has been rinsed, but not necessarily disaggregated.

In one system, system 10 includes a temperature control device that is positioned with respect to system 10 to adjust the temperature of the material contained in the tissue collection container 12. In certain systems, the temperature control device is a heater, and in other embodiments, temperature control device is a cooler. In additional systems, the temperature control device may be able to switch between a heater and a cooler. The temperature control device may be a device that adjusts the temperature of the adipose tissue contained in tissue collecting container 12, or may be a device that is positioned to change the temperature of fluid being delivered to tissue collecting container 12. It has been found that heating the adipose tissue facilitates disaggregation of the tissue to enhance the separation of the active cell component. In addition, it is desirable in certain systems to cool a portion of the tissue, preferably the active cell component to provide protection to the cells. Even mild cooling of the cells may provide suitable protection to enhance cell survival during the processing.

Outlet 32 of tissue removal system 10 is illustrated as being a component of mixing container 30. In additional systems, outlet 32 is spaced apart from mixing container 30. Outlet 32 preferably comprises a closure that maintains the sealed configuration of tissue removal system 10, and in certain systems, outlet 32 comprises a fluid impermeable membrane (e.g., a membrane that is impermeable to liquid and air). Outlet 32 should be structured to pass the composition in mixing container 30 to a patient under the appropriate conditions. For example, if a syringe is used to withdraw the composition, outlet 32 should be able to accommodate a needle of the syringe without compromising the sterility of the system or composition. In additional systems, if the outlet is coupled to a device that is configured to administer the composition, but not to withdraw the composition, such as a cannula that administers the composition by applying positive pressure to displace the composition through the cannula, outlet 32 should be configured to allow the composition contained in mixing container 30 to be passed into the cannula. In other versions of the system, outlet 32 may comprise, or be coupled in a closed-system fashion to, the device for administering the composition, such as a needle of a syringe or a cannula for administering the composition by applying positive pressure.

Tissue removal system 10 is also illustrated as including a waste container 36 positioned to collect waste from tissue collection container 12. In the illustrated system, waste container 36 is also coupled and positioned to receive waste from cell collection container 26. A wash container 38 is provided in fluid communication with wash line 39 to deliver a washing fluid, such as saline or any other suitable buffer, via wash port 46 to tissue collection container 12. Tissue collection container 12 also includes an air inlet 40 for controlling the amount of pressure within tissue collection container 12. An additive line 42 is provided on tissue collection container 12 to permit an additive to be added to tissue collection container 12. In reference to the methods disclosed herein, additive line 42 is provided to deliver one or more enzymes to tissue collection container 12 to facilitate the separation of the active cell component from the rest of the adipose tissue contained in filter 16. As illustrated, additive line 42 comprises a needle 44 which can be used to receive the enzyme from a suitable container.

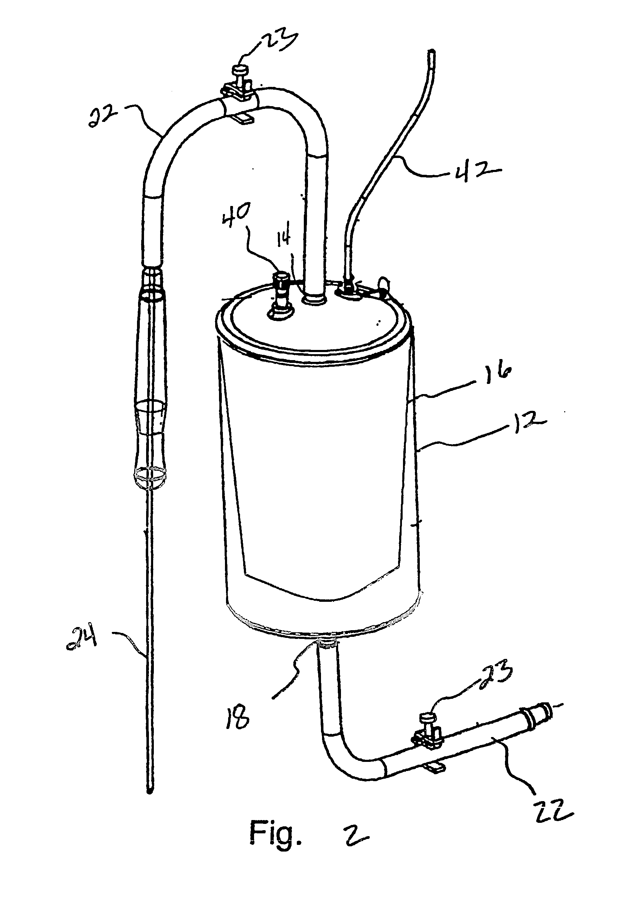

A particular version of the components of tissue removal system 10 are illustrated in Figs. 2 and 3 where like numbers represent like parts. In the particular system of Figs. 2 and 3, tissue collection container 12 includes a body that retains its form when suction is applied to the container. More specifically, tissue collection container 12 includes a rigid body, for example, a body constructed of a medical grade polycarbonate containing a roughly conical filter pocket of medical grade polyester with a mesh size of 275 µm. The rigid tissue collection container may have a size of approximately eight inches high and approximately five inches in diameter; the wall thickness may be about 0.125 inches. The interior of the cylinder is accessed through two ports for suction tubing, two ports with tubing for connection through sterile docking technology, and two ports for needle puncture access through a rubber septum. The same functionality could be achieved with different materials, mesh size, and the number and type of ports. For example, mesh pore sizes smaller than 100 µm or as large as several thousand microns would achieve the same purpose of allowing passage of saline and blood cells while retaining adipose tissue aggregates and fragments. Similarly, the device purpose could be achieved by use of an alternative rigid plastic material, by substitution of the disposable cannula with a non-disposable, multi-use sterile cannula, or by many, other modifications that would be known to those skilled in the art However, in other versions of tissue removal system 10, tissue collection container 12 may include a collapsible body, such as a tissue collection bag. In such systems, the bag is preferably provided with a support, such as an internal or external frame, that helps reduce the likelihood that the bag will collapse upon the application of suction to the bag.

In order to reduce contamination within tissue removal system 10, one or more clamps 23 may be provided on the various lines or conduits to control the flow of material through the lines to the various components of the system. Clamps 23 permit a user to effectively seal various regions of tissue removal system 10. In a preferred systems, one or more of the components of system 10 are disposable. Avoiding reusing the components in this embodiment helps to reduce contamination that may be associated with repeated use of various components. In addition, providing the components in a disposable set provides an advantage of being able to sterilize all of the components at a single time, which may substantially reduce the time required for practicing the methods disclosed herein. In fully or partially automated versions of the system, computer-controlled valves may be implemented in addition to or as an alternative to clamps 23.

In addition, tissue removal system 10 may include additional devices or components that permit, among other things, determination of the volume of material retained in the filter 16, to allow recording of written information regarding the extraction or processing procedure, or perform other supplementary functions such as attaching the device to a stand or bedding during operation.

The components of the tissue removal system 10 should be made of materials that are non-reactive with biological fluids or tissues, and non-reactive with agents used in processing biological fluids and tissues. In addition, the materials from which the various components are made should be capable of withstanding sterilization, such as by autoclaving, and irradiation, including but not limited to beta- or gamma-irradiation. The tubing and the cannula handle may be made of any suitable material, such as polyethylene. The cannula may be made of any suitable material, including stainless steel.

In accordance with the invention herein disclosed, the tissue removal system 10 provides a closed system that is convenient for removal, processing, and administration of adult stem cells found in adipose tissue. The system can be placed near the patient for removal of adipose tissue, and the tissue can be processed without requiring the tissue to be removed from the system. Thus, a system is disclosed that can provide fresh stem cell enhanced compositions to a patient, and reduces potential risks associated with culturing and or preserving stem cells.

Accordingly, based on the instant disclosure, the present invention may use a method for extracting tissue from a patient using the following steps: (i) preparing the patient as for traditional lipoplasty; (ii) removing the cannula and the tissue removal system from the packaging materials to the sterile field; (iii) connecting a liposuction pump (with conventional trap and in-line microbial filters) to the hose adaptor leading from the tissue collection container; (iv) ensuring that the tubing screw clamps are not engaged on the suction ports of the tissue collection container; (v) using the cannula as a regular liposuction cannula to remove unwanted adipose tissue; (vi) applying in a manual operation embodiment two tubing screw clamps to seal the tissue collection container after the desired amount of adipose tissue have been collected with the tissue collection container; (vii) ensuring that the tissue collection container is properly labeled with a patient identification label, and recording other information on the label (date and time of procedure, etc.) in accordance with institutional practice, and (viii) extracting adipose tissue from the patient.

Referring to the illustrated tissue removal system 10, tissue is collected directly into the processing components by attaching the tubing 22 to the suction source 20 with an in-line fluid trap and inserting the cannula 24 into the harvest site. Adipose tissue is then aspirated into the tissue collecting container 12 where it is retained by the filter 16 held within the tissue collection container 12. Following tissue collection the collected adipose tissue can be rinsed with a washing fluid, such as sterile isotonic saline, contained in wash container 38 added to tissue collection container 12 via wash line 39. When the tissue collecting container 12 is made of a rigid material in the illustrated embodiment to support collection under suction, the air displaced from the housing during addition of saline can be vented through the air-inlet port 40. Alternatively the air may be displaced into the waste container 36 or similar holding place. Once the tissue is rinsed the waste material can be allowed to flow into the waste container 36.

After the tissue has been collected, needle 44 can be inserted into a sterile vial of collagenase-containing enzyme solution which is then passed into tissue collection container 12 where it is mixed with the adipose tissue at or around 37° C for 15-60 minutes. Washing steps may be repeated as needed and the disaggregated tissue may be washed following elution of the active cell population in order to maximize yield. At the end of tissue disaggregation the tissue collection container 12 is placed upright to allow flotation of the adipocytes. The active cell population is then allowed to flow into cell collection container 26 where the cells are separated from collagenase and residual free lipid. Cells may be washed and/or concentrated by any method known to persons of ordinary skill in the art including but not limited to sequential centrifugation/re-suspension washes or continuous flow mechanisms. The concentrated, washed cells are then allowed to flow into mixing container 30 where they can be mixed with intact tissue from tissue retrieval line 34 and/or any intended additives before being removed through the outlet 32 for administration to a patient. The material contained in cell collecting container 26 may be filtered using cell filter 28 following washing to enhance removal of unwanted residual cell and tissue aggregates that could lead to embolism upon application.

During the processing, one or more additives may be added to the various containers as needed to enhance the results. Some examples of additives include agents that optimize washing and disaggregation, additives that enhance the viability of the active cell population during processing, anti-microbial agents (e.g., antibiotics), additives that lyse adipocytes and/or red blood cells, or additives that enrich for cell populations of interest (by differential adherence to solid phase moieties or to otherwise promote the substantial reduction or enrichment of cell populations).

In the above system, the tissue collecting container 12 is intrinsic to the processing components of the tissue removal system 10. Alternatively a separate tissue collecting container, such as that described in Patent Application No. 10/242,094, entitled PRESERVATION OF NON EMBRYONIC CELLS FROM NON HEMATOPOIETIC TISSUES, filed September 12, 2002, which claims the benefit of U.S. Provisional Patent Application 60/322,070 filed September 14, 2001, could be employed in whole or in part with subsequent transference of the disaggregated material to the processing components. Additional potential tissue collecting containers are disclosed in U.S. Patent Nos. 6,316,247 and 5,372,945.

As indicated above, the methods may be automated by providing one or more additional devices that can automatically perform the steps of the methods. In such implementations, a processing device (e.g., microprocessor or personal computer) is a device to partially or completely automate the steps described above. Examples of steps amenable to such automation include, but are not limited to, controlling the ingress and egress of fluids and tissues along particular tubing paths by controlling pumps and valves of the system or processing device; detecting blockages with pressure sensors; mixing mechanisms, measuring the amount of tissue and/or fluid to be moved along a particular pathway using volumetric mechanisms; maintaining temperatures of the various components using heat control devices; washing and concentrating the cell, and integrating the process with timing and software mechanisms. In one system, software can control the parameters of the process to allow production of a cell population prepared to specific operator-defined parameters. Thus, the automation device or devices improve the performance of the procedures, and provide automatic harvesting of adipose tissue and processing of the adipose tissue for administration to a patient.

One particular automation device is illustrated in Fig. 4. A tissue removal container (not shown) is placed into a device 100 using color-coded guide marks 112-118 to properly align and insert the tubing into appropriate paths. Device 100 includes a plurality of valves 105 and 110, and a plurality of pumps 104 and 109. Tubing is placed into a series of valves 105, 110 and pumps 104, 109 which are controlled by an integrated microprocessor system to coordinate fluid and tissue flow in accordance with the user defined program. Program selection is mediated through a user interface panel 106. A saline container is placed onto a holding structure 101 and attached to the tissue collection container. A vial or tube of collagenase or other tissue dissociation medium or mixture (not shown) is inserted into the tissue collection container at point 103. A waste bag (not shown) is inserted into a holding structure 111, the cell separation chamber/cell collection container is placed into a holding structure 107, and the tissue/cell mixing container is placed into the holding structure 108. The tissue collection container is placed into the agitation/incubation chamber 102.

Adipose tissue may be collected into the tissue collecting container while the container is in position within the device or prior to placement within the device. The device may contain an optional transparent insert 119 or other device allowing determination of the volume of tissue within the tissue collecting container. Alternatively volume may be determined by measurement of the weight of material contained in the agitation/incubation chamber 102 (corresponding to tissue collecting container 12). This volume may be displayed on the user interface screen 106.

The microprocessor then opens the valves 105 on lines 114 and 115 and activates the pumps 104 on line 114 for introduction of saline into the collection chamber 102 and removal of waste material 115 to the waste bag 111. During this process the collection chamber is agitated by rocking, and is maintained at a programmed temperature by warming devices integrated into the chamber 102. In certain systems, tissue processing may use prewarmed saline in which case the role of the warming device of the agitation/incubation chamber is to maintain temperature at the determined preprogrammed point rather than to increase the temperature.

Once the tissue is washed some fraction from 0% to 100% of the intact, washed adipose tissue may be removed from the incubation chamber 102 by activation of the pump 109 and valve 110 on line 116. Material withdrawn at this time is held in the mixing chamber 108. Dissociation medium 103 is added to material remaining in the chamber 102 by opening the valve 105 on line 113, closing other valves and activating pump 104 on line 113. After addition of dissociation medium the chamber 102 is agitated and maintained at temperature as described above. At the conclusion of the programmed incubation period agitation is halted to allow flotation of adipocytes. Additional saline may be added to facilitate this process. Following flotation of adipocytes, the valves on lines 112 and 115 are opened to allow removal of the target cell population from the chamber 102 into the cell washing chamber 107. Washed cells are removed through line 117 into the mixing chamber 108, supernatant and washing solution are removed into the waste chamber 111 through line 118. Additional saline is passed into the system through line 114 to complete the washing process. Cells are mixed in the chamber 108 with any intact tissue removed through line 116 earlier in processing. Mixing may be achieved by any means know to those skilled in the art including but not limited to agitation rocking/inversion of chamber, or by compression pulsed or by moving rollers. Mixed material may then be removed through the port in the mixing chamber of the disposable set.

The device includes a microprocessor-controlled mechanism for automating the process according to pre-programmed parameters 106. This system would also include use of pressure sensors for detection of blockages and similar safety and quality control mechanisms. In a preferred system the software component of the system would include automated collection of "run data" including, for example, the lot numbers of disposable components, temperature and volume measurements, tissue volume and cell number parameters, dose of enzyme applied, incubation time, operator identity, date and time, patient identity, etc.. In a preferred embodiment of the device a bar code reading system would be integrated to permit data entry of these variables (for example disposable set lot number and expiration date, lot number and expiration date of the Collagenase, patient/sample identifiers, etc.) into the device controller as part of documentation of processing. This would reduce the opportunity for data entry errors. This device could be easily incorporated into the controller system using a USB or other interface port and system known to the art. In this way the device would provide integrated control of the data entry and documentation of the process. A print-out report of these parameters would be part of the user-defined parameters of a programmed operation of the device. Naturally this would require integration of a printer component (hardware and driver) or printer driver in software plus an interface output connector for a printer (e.g., a USB port) in the hardware of the device.

In a further system, software incorporated into the controller would prompt users through the steps necessary for proper insertion of tubing and other elements into the device. Software would also initiate automated testing to confirm correct insertion of tubing, absence of blockages, etc.

The general approach to processing in this device would use the same parameters as those described elsewhere in this disclosure for manual cell processing.

Many other conformations of the staged mechanisms used for cell processing will be apparent to one skilled in the art and the present description is included as one example only. For example, mixing of tissue and saline during washing and disaggregation may occur by agitation as in the present example or by fluid recirculation. Cell washing may be mediated by a continuous flow mechanism such as the spinning membrane approach, differential adherence, differential centrifugation (including, but not limited to differential sedimentation, velocity, or gradient separation), or by a combination of means. Similarly, additional components to allow further manipulation of cells including addition of growth factors or other biological response modifiers (Lind, 1998) (Hanada et al., 1997) (Lieberman et al., 1998), mixing of cells with natural or synthetic components intended for implant with the cells into the recipient (Fukuda, 2001; Sodian et al., 2002; Ye et al., 2000).

Post-processing manipulation may also include cell culture (Caplan and Bruder, 2001; De Ugarte et al., 2003; Zuk et al., 2001), gene transfer (Luskey et al., 1990; Morizono et al., 2003), or further cell purification (Greenberg and Hammer, 2001; Mainwaring and Rowley, 1985; Schweitzer et al., 1995). Mechanisms for performance of such functions may be integrated within the device shown in Figure 4 or may be incorporated in separate devices.

In additional system, tissue collected into a conventional adipose tissue trap could be transferred into a processing set designed for processing other tissues. For example, Baxter Inc. manufacture and sell a series of plastic bags and filters intended for use in the setting of a bone marrow transplant harvest ("Bone Marrow Collection Kit with Flexible Pre-Filters and Inline Filters", Product Code, 4R2107, U.S. Pat. Nos. 4,346,703 and 5,724,988). This bag set contains a large conical bag with an integrated 800µm filter which could be used for washing the collected adipose tissue. In this example adipose tissue fragments larger than 800µm would be retained in the bag. These fragments could then be washed by repeated addition of saline (or other washing solution) followed by removal of waste material through ports below the filter. Mixing could be achieved manually or by use of a bench top rocking device and warming could be applied by use of a heating pad. Disaggregation could occur within the lumen of this bag. Following disaggregation cells would pass through the integrated 800µm filter (and optionally through one or more filters of smaller mesh size provided with the kit) and collected into a collection bag (also provided). This bag could then be placed into a centrifuge (e.g., a Sorval RC-3C) where cells could be serially washed and concentrated. Cells could also be washed using existing cell washing devices (largely developed for washing human blood products) such as those sold by Baxter Inc (Cytomate or Baxter CS3000) or by Cobe Inc. (Cobe Spectra). The disposable elements may be integrated using the fittings provided by the manufacturer or they may be linked by use of a sterile connecting device such as those manufactured by Terumo Inc. Similarly the mechanisms described in this less integrated approach could be linked to a central controller and assembled as components of a more integrated device. A peristaltic pump or battery of pumps could be used to automate fluid flow with use of manual or automated clamping to open and close fluid pathways.

In a preferred version, the tissue removal system and processing set would be present in the vicinity of the patient receiving the treatment, such as the operating room or out-patient procedure room (effectively at the patient's bedside). This allows rapid, efficient tissue harvest and processing, remove the opportunity for specimen handling/labeling error and thereby allow for performance of the entire process in the course of a single surgical procedure.

The following examples are provided to demonstrate particular situations and settings in which this technology may be applied and are not intended to restrict the scope of the invention and the claims included in this disclosure.

### 2. Methods of Treating Cardiovascular Diseases and Disorders Using Processed Lipoaspirate (ADC)

As demonstrated in the instant disclosure, in a particularly preferred embodiment, the ADC of the invention can be used to treat cardiovascular diseases and disorders. Adipose tissue-derived stem and progenitor cells as obtained by practicing the methods used by the invention invention have several properties that can contribute to reducing and/or minimizing damage and promoting myocardial or cardiovascular repair and regeneration following damage. These include, among other things, the ability to synthesize and secrete growth factors stimulating new blood vessel formation, the ability to synthesize and secrete growth factors stimulating cell survival and proliferation, the ability to proliferate and differentiate into cells directly participating in new blood vessel formation, the ability to engraft damaged myocardium and inhibit scar formation (collagen deposition and cross-linking), the ability to proliferate and differentiate into muscle cells capable of contributing to myocardial contractility, and the ability to proliferate and differentiate into myocardial cells.

The foregoing means for reducing and/or minimizing damage and promoting myocardial or cardiovascular repair and regeneration following damage using the adipose derived adult stem cells of the invention are described in detail below in the Examples portion of the instant disclosure. Specifically, the present invention demonstrates, for the first time, that the adipose derived stem cells (or ADC) of the invention express numerous angiogenic growth factors, including Placenta Growth Factor (PIGF) and Vascular Endothelial Growth Factor (VEGF), contain endothelial progenitor cells (EPC) which have a well-established function in blood vessel formation, develop into blood vessels *in vitro*, support ischemic tissue survival in vivo, induce reperfusion following occlusion/reperfusion injury of the hind limb, home to the heart when injected into animals after heart injury, and differentiate into cells expressing markers consistent with their differentiation into cardiac myocytes when injected into an animals after heart injury.

Accordingly, adipose tissue-derived cells are obtained from a donor's adipose tissue and are used to elicit a therapeutic benefit to damaged or degenerated myocardium or other cardiovascular tissue through one or more of the mechanisms demonstrated herein. In a preferred embodiment the cells are obtained from the adipose tissue of the person into whom they are to be implanted, thereby reducing potential complications associated with antigenic and/or immunogenic responses to the transplant. Patients are typically evaluated to assess myocardial damage or disease by one or more of the following procedures performed by a physician or other clinical provider: patient's health history, physical examination, and objective data including but not limited to EKG, serum cardiac enzyme profile, and echocardiography.

The harvesting procedure may be performed prior to the patient receiving any products designed to reduce blood clotting in connection with treatment of the myocardial infarction. However, in certain embodiments, the patient may have received aspirin prior to the harvesting procedure. In addition, collection of adipose tissue have occured prior to any attempted revascularization procedure. However, as understood by persons skilled in the art, the timing of collection is expected to vary and will depend on several factors including, among other things, patient stability, patient coagulation profile, provider availability, and quality care standards. Ultimately, the timing of collection will be determined by the practitioner responsible for administering care to the affected patient.

The volume of adipose tissue collected from the patient can vary from about 0 cc to about 2000 cc and in some embodiments up to about 3000 cc. The volume of fat removed will vary from patient to patient and will depend on a number of factors including but not limited to: age, body habitus, coagulation profile, hemodynamic stability, severity of infarct, co-morbidities, and physician preference.

Cells may be for administration to a patient in any setting in which myocardial function is compromised. Examples of such settings include, but are not limited to, acute myocardial infarction (heart attack), congestive heart failure (either as therapy or as a bridge to transplant), and supplementation of coronary artery bypass graft surgery, among other things. The cells may be extracted in advance and stored in a cryopreserved fashion or they may be extracted at or around the time of defined need. As disclosed herein, the cells may be for administration to the patient, or for application directly to the damaged tissue, or in proximity of the damaged tissue, without further processing or following additional procedures to further purify, modify, stimulate, or otherwise change the cells. For example, the cells obtained from a patient may be for administration to a patient in need thereof without culturing the cells before administering them to the patient. In one embodiment, the collection of adipose tissue was performed at a patient's bedside. Hemodynamic monitoring may be used to monitor the patient's clinical status.

In accordance with the invention herein disclosed, the adipo-derived cells can be for delivery to the patient soon after harvesting the adipose tissue from the patient. For example, the cells may be for administration immediately after the processing of the adipose tissue to obtain a composition of adipo-derived stem cells. In one embodiment, the preferred timing of delivery should take place on the order of hours to days after the infarction to take advantage of the neurohormonal environment which exists after cardiac injury. Ultimately, the timing of delivery will depend upon patient availability and the processing time required to process the adipose tissue. In another embodiment, the timing for delivery may be relatively longer if the cells to be re-infused to the patient are subject to additional modification, purification, stimulation, or other manipulation, as discussed herein. Furthermore, adipo-derived cells may be for administration multiple times after the infarction. For example, the cells may be administered continuously over an extended period of time (e.g., hours), or may be administered in multiple bolus injections extended over a period of time. In certain embodiments, an initial administration of cells will be administered within about 12 hours after an infarction, such as at 6 hours, and one or more doses of cells will be administered at 12 hour intervals.

The number of cells administered to a patient may be related to, for example, the cell yield after adipose tissue processing. A portion of the total number of cells may be retained for later use or cyropreserved. In addition, the dose delivered will depend on the route of delivery of the cells to the patient. Fewer cells may be needed when epicardial or endocardial delivery systems are employed, as these systems and methods can provide the most direct pathway for treating cardiovascular conditions. In one embodiment of the invention, a number of cells, e.g., unpurified cells, to be delivered to the patient is expected to be about 5.5 x 10⁴ cells. However, this number can be adjusted by orders of magnitude to achieve the desired therapeutic effect.

The cells may also be applied with additives to enhance, control, or otherwise direct the intended therapeutic effect. For example, in one embodiment, and as described herein, the cells may be further purified by use of antibody-mediated positive and/or negative cell selection to enrich the cell population to increase efficacy, reduce morbidity, or to facilitate ease of the procedure. Similarly, cells may be applied with a biocompatible matrix which facilitates *in vivo* tissue engineering by supporting and/or directing the fate of the implanted cells. In the same way, cells may be for administration following genetic manipulation such that they express gene products that are believed to or are intended to promote the therapeutic response(s) provided by the cells. Examples of manipulations include manipulations to control (increase or decrease) expression of factors promoting angiogenesis or vasculogenesis (for example VEGF), expression of developmental genes promoting differentiation into specific cell lineages (for example MyoD) or that stimulate cell growth and proliferation (for example bFGF-1).

The cells may also be subjected to cell culture on a scaffold material prior to being implanted. Thus, tissue engineered valves, ventricular patches, pericardium, blood vessels, and other structures could be synthesized on natural or synthetic matrices or scaffolds using ADC prior to insertion or implantation into the recipient (Eschenhagen et al., 2002; Zimmermann et al., 2004; Zimmermann et al., 2002; Nerem and Ensley, 2004). Indeed, *in vitro* differentiation of adipose derived stem and progenitor cells into cells expressing markers of cardiac myocytes has been demonstrated (Gaustad et al., 2004; Rangappa et al., 2003).

### 3. Routes of Administration of the ADC for the Treatment of Cardiovascular Disease and Disorders

In one embodiment, direct administration of cells to the site of intended benefit is preferred. This may be achieved by direct injection into the external surface of the heart (epicardial), direct injection into the myocardium through the internal surface (endocardial) through insertion of a suitable cannula, by arterial or venous infusion (including retrograde flow mechanisms) or by other means disclosed herein or known in the art. Routes of administration known to one of ordinary skill in the art, include but are not limited to, intravenous, intracoronary, endomyocardial, epimyocardial, intraventicular, retrosinus or intravenous.

As mentioned above, cells may for administration by several routes including systemic administration by venous or arterial infusion (including retrograde flow infusion) or by direct injection into the heart. Systemic administration, particularly by peripheral venous access, has the advantage of being minimally invasive relying on the natural perfusion of the heart and the ability of adipose tissue-derived cells to target the site of damage. Cells may be for injection in a single bolus, through a slow infusion, or through a staggered series of applications separated by several hours or provided cells are appropriately stored, several days or weeks. Cells may also be for administration by use of catheterization such that the first pass of cells through the heart is enhanced by using balloons to manage myocardial blood flow. As with peripheral venous access, cells may be injected through the catheters in a single bolus or in multiple smaller aliquots. Cells may also be for application directly to the myocardium by epicardial injection. This could be employed under direct visualization in the context of an open heart procedure (such as a Coronary Artery Bypass Graft Surgery) or placement of a ventricular assist device. Catheters equipped with needles may be employed to deliver cells directly into the myocardium in an endocardial fashion which would allow a less invasive means of direct application.

In one embodiment, the route of delivery will include intravenous delivery through a standard peripheral intravenous catheter, a central venous catheter, or a pulmonary artery catheter. In other embodiments, the cells may be delivered through an intracoronary route to be accessed via currently accepted methods. The flow of cells may be controlled by serial inflation/deflation of distal and proximal balloons located within the patient's vasculature, thereby creating temporary no-flow zones which promote cellular engraftment or cellular therapeutic action. In another embodiment, cells may be delivered through an endocardial (inner surface of heart chamber) method which may require the use of a compatible catheter as well as the ability to image or detect the intended target tissue. Alternatively, cells may be delivered through an epicardial (outer surface of the heart) method. This delivery may be achieved through direct visualization at the time of an open heart procedure or through a thoracoscopic approach requiring specialized cell delivery instruments. Furthermore, cells could be for delivery through the following routes, alone, or in combination with one or more of the approaches identified above: subcutaneous, intramuscular, sublingual, retrograde coronary perfusion, coronary bypass machinery, extracorporeal membrane oxygenation (ECMO) equipment and via a pericardial window.

In one embodiment, cells are for administration to the patient as an intra-vessel bolus or timed infusion. In another embodiment, cells may be resuspended in an artificial or natural medium or tissue scaffold prior to be administered to the patient.

The cell dose to be administered to the patient will be dependent on the amount of adipose tissue harvested and the body mass index of the donor (as a measure of the amount of available adipose tissue). The amount of tissue harvested will also be determined by the extent of the myocardial injury or degeneration. Multiple treatments using multiple tissue harvests or using a single harvest with appropriate storage of cells between applications are within the scope of this invention.

Portions of the processed lipoaspirate may be stored before being administered to a patient. For short term storage (less than 6 hours) cells may be stored at or below room temperature in a sealed container with or without supplementation with a nutrient solution. Medium term storage (less than 48 hours) is preferably performed at 2-8°C in an isosmotic, buffered solution (for example Plasmalyte®) in a container composed of or coated with a material that prevents cell adhesion. Longer term storage is preferably performed by appropriate cryopreservation and storage of cells under conditions that promote retention of cellular function, such as disclosed in commonly owned and assigned PCT application number PCT/US02/29207, filed September 13, 2002 and U.S. Provisional application number 60/322,070, filed September 14, 2001.

In accordance with one aspect of the invention, the adipose-tissue derived cells that are to be administered to a patient can act as growth factor delivery vehicles. For example, by engineering the cells to express one or more growth factors suitable for alleviating symptoms associated with a cardiovascular disorder or disease, the cells can be administered to a patient, and engineered to release one or more of the growth factors. The release can be provided in a controlled fashion for extended periods of time. For example, the release can be controlled so that the growth factor(s) are released in a pulsed or periodic manner such that there are local elevations in growth factor, and/or local recessions in the amount of growth factor in proximity to an injured area of tissue.

The cells that are to be administered to the patient not only help restore function to damaged or otherwise unhealthy tissues, but also facilitate remodeling of the damaged tissues.

Cell delivery may take place but is not limited to the following locations: clinic, clinical office, emergency department, hospital ward, intensive care unit, operating room, catheterization suites, and radiologic suites.

In one embodiment, the effects of cell delivery therapy would be demonstrated by, but not limited to, one of the following clinical measures: increased heart ejection fraction, decreased rate of heart failure, decreased infarct size, decreased associated morbidity (pulmonary edema, renal failure, arrhythmias) improved exercise tolerance or other quality of life measures, and decreased mortality. The effects of cellular therapy can be evident over the course of days to weeks after the procedure. However, beneficial effects may be observed as early as several hours after the procedure, and may persist for several years.

Patients are typically monitored prior to and during the deliver of the cells. Monitoring procedures include, and are not limited to: coagulation studies, oxygen saturation, hemodynamic monitoring, and cardiac rhythm monitoring. After delivery of cells, patients may require an approximate 24 hour period of monitoring for adverse events. Follow-up studies to assess functional improvements from the procedures may include and are not limited to: patient functional capacity (e.g., dyspnea on exertion, paroxysmal nocturnal dysnpea, angina), echocardiography, nuclear perfusion studies, magnetic resonance imaging, postiron emission topography, and coronary angiography.

As previously set forth above, in a preferred embodiment, the ADC, *i.e.*, the active adipose derived stem cell population, is to be administered directly into the patient. In other words, the active cell population (e.g., the stem cells and/or endothelial precursor cells) are to be administered to the patient without being removed from the system or exposed to the external environment of the system before being administered to the patient. Providing a closed system reduces the possibility of contamination of the material being administered to the patient. Thus, processing the adipose tissue in a closed system provides advantages over existing methods because the active cell population is more likely to be sterile. In such an embodiment, the only time the stem cells and/or endothelial precursor cells are exposed to the external environment, or removed from the system, is when the cells are being withdrawn into an application device and being administered to the patient. In one embodiment, the application device can also be part of the closed system. Thus, the cells used in these embodiments are not processed for culturing or cryopreservation and may be for administration to a patient without further processing, or to be administered to a patient after being mixed with other tissues or cells.

In other embodiments, at least a portion of the active cell population is stored for later implantation/infusion. The population may be divided into more than one aliquot or unit such that part of the population of stem cells and/or endothelial precursor cells is retained for later application while part is applied immediately to the patient. Moderate to long-term storage of all or part of the cells in a cell bank is also within the scope of this invention, as disclosed in U.S. Patent Application No. 10/242,094, entitled PRESERVATION OF NON EMBRYONIC CELLS FROM NON HEMATOPOIETIC TISSUES, filed September 12, 2002, which claims the benefit of U.S. Provisional Patent Application 60/322,070 filed September 14, 2001. At the end of processing, the concentrated cells may be loaded into a delivery device, such as a syringe, for placement into the recipient by any means known to one of ordinary skill in the art.

### II. Pharmaceutical Compositions

The active cell population may be applied alone or in combination with other cells, tissue, tissue fragments, growth factors such as VEGF and other known angiogenic or arteriogenic growth factors, biologically active or inert compounds, resorbable plastic scaffolds, or other additive intended to enhance the delivery, efficacy, tolerability, or function of the population. The cell population may also be modified by insertion of DNA or by placement in cell culture in such a way as to change, enhance, or supplement the function of the cells for derivation of a structural or therapeutic purpose. For example, gene transfer techniques for stem cells are known by persons of ordinary skill in the art, as disclosed in (Morizono et al., 2003; Mosca et al., 2000), and may include viral transfection techniques, and more specifically, adeno-associated virus gene transfer techniques, as disclosed in (Walther and Stein, 2000) and (Athanasopoulos et al., 2000). Non-viral based techniques may also be performed as disclosed in (Muramatsu et al., 1998).

In another aspect, the cells could be combined with a gene encoding pro-angiogenic and/or cardiomyogenic growth factor(s) which would allow cells to act as their own source of growth factor during cardiac repair or regeneration. Genes encoding anti-apoptotic factors or agents could also be applied. Addition of the gene (or combination of genes) could be by any technology known in the art including but not limited to adenoviral transduction, "gene guns," liposome-mediated transduction, and retrovirus or lentivirus-mediated transduction, plasmid, adeno-associated virus. Cells could be implanted along with a carrier material bearing gene delivery vehicle capable of releasing and/or presenting genes to the cells over time such that transduction can continue or be initiated *in situ*. Particularly when the cells and/or tissue containing the cells are to be administered to a patient other than the patient from whom the cells and/or tissue were obtained, one or more immunosuppressive agents may be administered to the patient receiving the cells and/or tissue to reduce, and preferably prevent, rejection of the transplant. As used herein, the term "immunosuppressive drug or agent" is intended to include pharmaceutical agents which inhibit or interfere with normal immune function. Examples of immunosuppressive agents suitable with the methods disclosed herein include agents that inhibit T-cell/B-cell costimulation pathways, such as agents that interfere with the coupling of T-cells and B-cells via the CTLA4 and B7 pathways, as disclosed in U.S. Patent Pub. No. 20020182211. A preferred immunosuppressive agent is cyclosporine A. Other examples include myophenylate mofetil rapamicin, and anti-thymocyte globulin. In one embodiment, the immunosuppressive drug is to be administered with at least one other therapeutic agent. The immunosuppressive drug is in a formulation which is compatible with the route of administration and is to be administered to a subject at a dosage sufficient to achieve the desired therapeutic effect. In another embodiment, the immunosuppressive drug is to be administered transiently for a sufficient time to induce tolerance to the ADC of the invention.

In certain embodiments of the invention, the cells are for administration to a patient with one or more cellular differentiation agents, such as cytokines and growth factors. Examples of various cell differentiation agents are disclosed in (Gimble et al., 1995; Lennon et al., 1995; Majumdar et al.,1998; Caplan and Goldberg,1999; Ohgushi and Caplan, 1999; Pittenger et al., 1999; Caplan and Bruder, 2001; Fukuda, 2001; Worster et al., 2001; Zuk et al., 2001).

The present invention is further illustrated by the following examples which in no way should be construed as being further limiting.

### EXAMPLES

The ADC or active population of adipose derived stem cells used throughout the examples set forth below were obtained by the method(s) described in the instant disclosure and/or the method(s) described in U.S. Application Serial No. 10/316,127, entitled SYSTEMS AND METHODS FOR TREATING PATIENTS WITH PROCESSED LIPOASPIRATE CELLS, filed December 9, 2002, which claims priority to U.S. Provisional Application Serial No. 60/338,856, filed December 7, 2001.

### EXAMPLE 1: Expression of Angiogenic Growth Factor, VEGF, by ADC

Vascular Endothelial Growth Factor (VEGF) is one of the key regulators of angiogenesis (Nagy et al., 2003; Folkman, 1995). Placenta Growth Factor, another member of the VEGF family, plays a similar role in both angiogenesis as well as in arteriogenesis, the process by which collateral vessels are recruited and expanded in response to increased perfusion and shear force (Nagy et al., 2003; Pipp et al., 2003; Scholz et al., 2003). Specifically, transplant of wild-type (PIGF +/+) cells into a P1GF knockout mouse restores ability to induce rapid recovery from hind limb ischemia (Scholz et al., 2003).

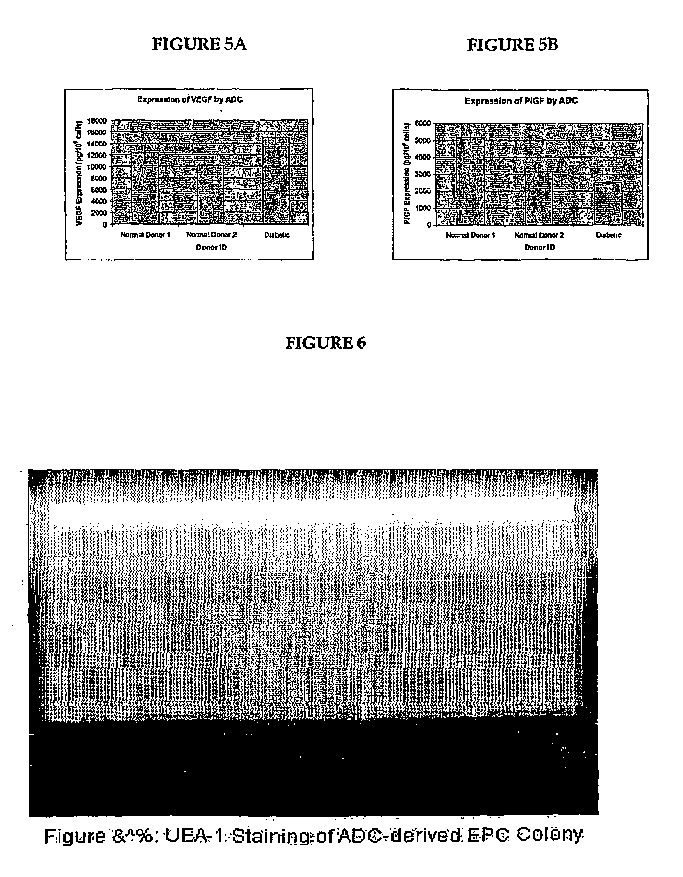

Given the importance of both angiogenesis and arteriogenesis to the revascularization process, PIGF and VEGF expression by ADC cells was examined using an ELISA assay (R&D Systems, Minneapolis, MN) using ADC cells from three donors. One donor had a history of hyperglycemia and Type 2 diabetes (a condition highly associated with microvascular and macrovascular disease, including patients with coronary artery disease). ADC cells from each donor were plated at 1,000 cells/cm² in DMEM/F-12 medium supplemented with 10% FCS and 5% HS and grown until confluent. Supernatant samples were taken and assayed for expression of PIGF and VEGF protein. As shown in Figures 5A and 5B, the results demonstrate robust expression of both VEGF (Figure 5A) and PIGF (Figure 5B) by the adipose derived stem cells of the invention.

These data demonstrate that adipose tissue derived stem and progenitor cells from both normal and diabetic patients express angiogenic and arteriogenic growth factors. This is important as patients with diabetes are at increased risk of cardiovascular disease and these data indicate that ADC cells retain their angiogenic ability in the diabetic setting. Thus, diabetic patients can derive angiogenic benefit from their own ADC cells.

### EXAMPLE 2: ADC Contains Cell Populations That Participate in Angiogenesis

Endothelial Progenitor Cells (EPCs) are known to participate in angiogenesis. Circulating endothelial precursor cells have been detected in peripheral blood, cord blood, marrow, and fetal liver (Takahashi, 1999; Asahara, 1999; Asahara, 1997; Loomans, 2004; Shintani, 2001; Vasa, 2001). To determine the frequency of EPCs in adipose derived stem cells, an EPC assay was performed. ADC cells were plated onto fibronectin-coated plates and cultured in endothelial cell medium for three days to remove mature endothelial cells. Nonadherent cells were removed and re-plated. After 14 days colonies were identified by staining with FITC-conjugated Ulex europaeus Agglutinin-1 (Vector Labs, Burlingame, CA) and DiI-labeled acetylated LDL (Molecular Probes, Eugene, OR). As shown in Figure 6, the results indicate an EPC frequency of approximately 500 EPC/10⁶ ADC cells.

The presence of EPCs within the adipose tissue derived stem and progenitor cell population indicates that this population can participate directly in development of new blood vessels and enhance angiogenesis and reperfusion, thereby reducing the duration of ischemia following myocardial infarction or in congestive heart failure.

### EXAMPLE 3: in Vitro Development of Vascular Structures in ADC

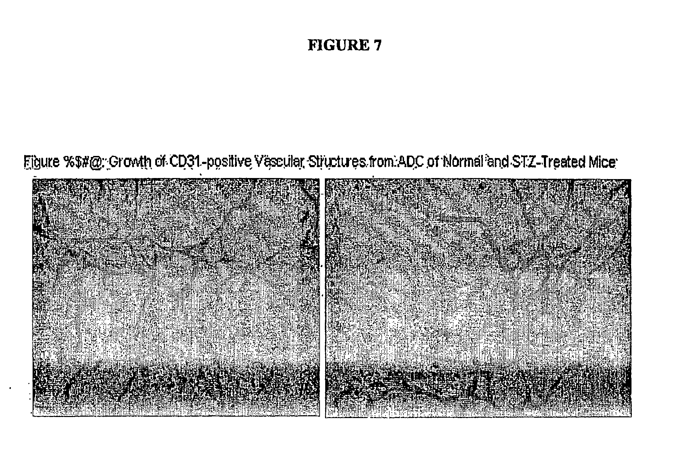

An art-recognized assay for angiogenesis is one in which endothelial cells grown on a feeder layer of fibroblasts develop a complex network of CD31-positive tubes reminiscent of a nascent capillary network (Donovan et al., 2001). ADC form similar networks *in the absence of a feeder layer* (Figure 7A). Notably, ADC cells obtained from hyperglycemic mice with streptozotocin (STZ)-induced Type 1 diabetes eight weeks following administration of STZ form similar structures at a similar frequency to those of untreated mice (Figure 7B).

This is important as patients with diabetes are at increased risk of cardiovascular disease and these data indicate that ADC cells retain their angiogenic ability in the diabetic setting. Thus, diabetic patients can derive angiogenic benefit from their own ADC cells.

In summary, the results of Examples 1 through 3, above, indicate that adipose derived stem cells contain populations of cells that can promote angiogenesis and arteriogenesis. The results also indicate that adipose derived stem cells from a diabetic human donor or from STZ-treated mice exhibiting sustained hyperglycemia are likely not deficient in cells that promote angiogenesis. Thus, adipose may represent a reservoir of regenerative cells that is not compromised by the diabetic setting in which risk of cardiovascular disease is substantially increased.

### EXAMPLE 4: In Vivo Development of Vascular Structures in ADC

*In vitro* angiogenic potential, while promising, is of little value if the cells do not exert *in vivo* angiogenic activity. Surgical induction of critical limb ischemia in rodents is a well-recognized model in which concurrent processes of arteriogenesis (recruitment and expansion of collateral vessels largely in response to increased shear force) and angiogenesis (development of new vessels in response to ischemia) can be observed {Schatteman, 2000; Scholz, 2002; Takahashi, 1999). This model was developed in immunodeficient (NOD-SCID) mice in which the ability of human cells to drive reperfusion could be observed. Specifically, animals were anesthetized with ketamine and xylazine (80mg.kg; 7.5mg/kg) and placed on the operating surface in the supine position. Pre-operative blood flow values were determined for both hind limbs as described below. Animals were prepped with Betadine and draped in the usual sterile fashion and placed on a circulating waterbath. A unilateral 1.5cm incision was made extending from the origin of the hind-limb to just proximal of the knee to expose the iliac artery, proximal to its bifurcation into the deep and superficial femoral arteries. The vasculature was tied off with a 3-0 silk ligature at the following sites: 1) iliac artery proximal to its bifurcation, 2) just distal to the origin of deep femoral artery, 3) just proximal to branching of the superficial femoral artery. After ligation, the vasculature was removed *en bloc.* An effort was then made to identify any obvious collaterals which were ligated and subsequently removed. The wound and the muscle layer were closed with 4-0 vicryl and the skin closed with 5-0 vicryl. Animals were treated post-operatively with buprenorphine (0.5mg/kg) and recovered on the circulating water bath until spontaneously recumbent. Twenty four hours after surgery animals were injected with 5x10⁶ ADC cells through the tail vein. NOD-SCID mice were injected with human donor cells, including in one study, cells from a patient with diabetes. Flow was imaged 14 days following treatment.

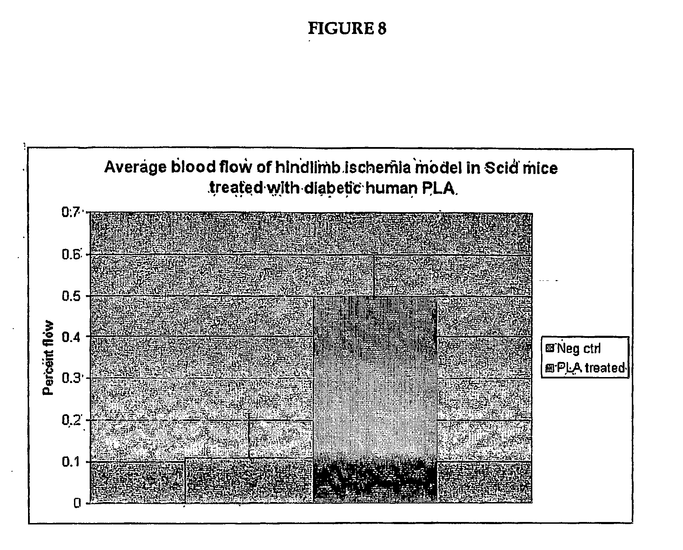

In these studies, ADC-treated animals showed statistically significant improvement in retention of limb structures (limb salvage; 2/3 untreated mice lost all lower hind limb structures compared with 0/5 ADC-treated animals) and restoration of flow (Figure 8). Most notably, in NOD SCID mice receiving diabetic human donor cells, day 14 flow was restored to 50±11% in treated animals compared to 10±10% in untreated animals (p<0.05). By day 19 rebound had occurred such that perfusion in the experimental limb was greater than that of the control (136±37%). This response is within the range observed with cells obtained from two normal (non-diabetic) donors (50-90%).

In a similar experiment in immunocompetent mice (129S mice) in which the effects of autologous cell transfer could be determined ADC cell treated mice exhibited 80±12% restoration of flow at day 14 compared to 56±4% in untreated mice.

In this model restoration of blood flow comes from the recruitment and expansion of collateral vessels and by angiogenesis in the lower limb. These processes also are key to restoration of flow in the heart following infarct. Thus, the ability of ADC to stimulate these processes *in vivo* strongly supports application of ADC cells in the setting of a myocardial infarction. It is also important to note that ADC cells obtained from a diabetic donor (a member of a patient population at higher risk of cardiovascular disease) also demonstrated this activity.

### EXAMPLE 5: Increasing ADC Dose Is Associated with Improved Graft Survival and Angiogenesis

Transplant of autologous adipose tissue is a relatively common procedure in plastic and reconstructive surgery {Fulton, 1998; Shiffman, 2001}. However, this procedure is limited by the fact that the adipose tissue fragments are transferred without a vascular supply and, as a result, graft survival is dependent upon neovascularization (Coleman, 1995; Eppley et al., 1990). Thus, in a limited way, the transplanted tissue represents an ischemic tissue.

A study in Fisher rats was performed in which adipose tissue fragments were transplanted into the subcutaneous space over the muscles of the outer thigh. The right leg was transplanted with 0.2g of adipose tissue fragments alone, the left leg with 0.2g of adipose tissue fragments supplemented by addition of adipose derived stem cells at three different doses (1.7x10⁵-1.3x10⁶ cells/graft; three animals per dose); in this way the contralateral leg acted as a control. Animals were then maintained for one month after which the animals were euthanized and the grafts recovered, weighed, fixed in formalin and embedded in paraffin for histologic analysis.

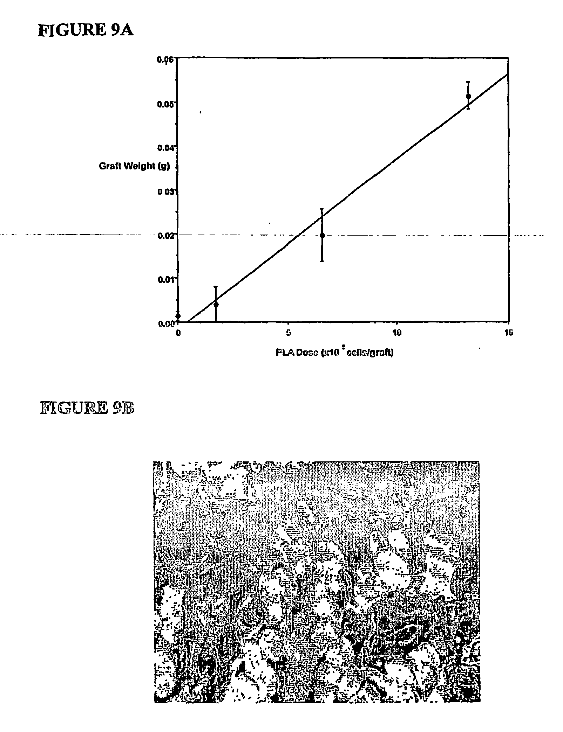

As shown in Figure 9A, the results show minimal retention of grafted tissue in the control leg and a dose-dependent increase in retention of graft weight in the treated leg. Further, immunohistochemical analysis of the grafts showed considerable neoangiogenesis and perfusion in the adipose derived stem cell treated grafts (Figure 9B, arrows). This was also associated with retention of adipose tissue morphology (Figure 9B).

As above, demonstration that ADC cells promote survival of inadequately perfused, ischemic tissue is an important indicator of clinical potential in cardiovascular disease.

### EXAMPLE 6: Myocardial-Engraftment by ADC

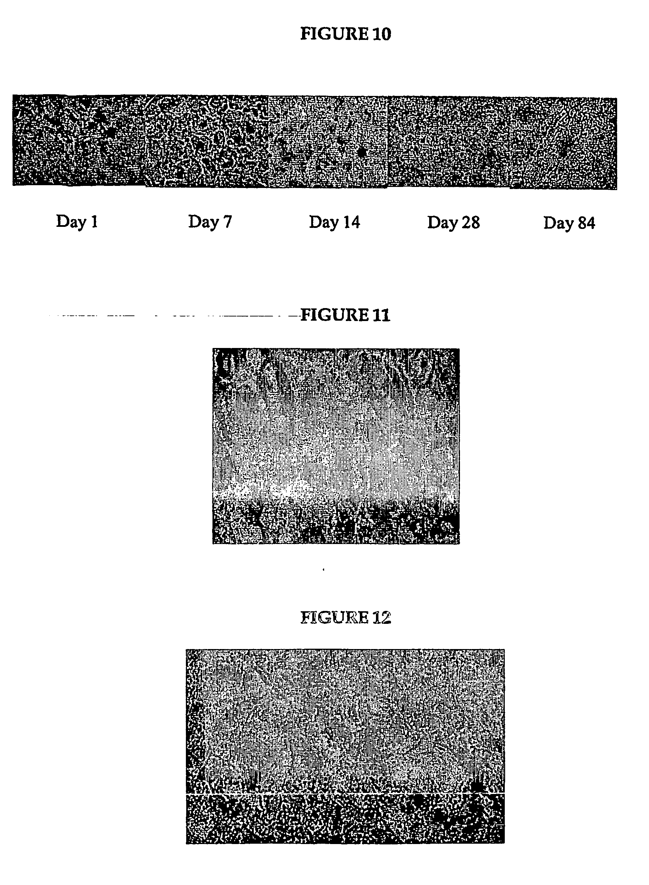

Cryoinjury to the myocardium is a well established surgical model to investigate the role of cellular therapy in myocardial regeneration (Marchlinski et al., 1987). To demonstrate the ability of ADC cells to engraft damaged myocardium and thereby inhibit scar formation (collagen deposition and cross-linking), myocardial cryoinjury in B6129SF1/J mice was performed. Immediately after injury, 1 million (1.0 x 10⁶) ADC cells harvested from ROSA26 mice which are transgenic for the lacZ gene were injected via an intra-ventricular route. Recipient heart tissue stained with B-galactosidase will detect the presence of donor adipose derived stem cells by staining blue. Mice hearts were harvested and processed at the following 5 time-points after injection: day 1, day 7, day 14, day 28, day 84. As shown in Figure 10, the results demonstrate engraftment of donor derived adipose derived stem cells in the area of infracted myocardium at all timepoints referenced above. Figure 10 demonstrates a histological timeline of engraftment.

Importantly, immunohistochemical analysis of donor-derived (beta galactosidase-positive) cells at day 14 indicated that many donor-derived cells expressed the cardiac myocyte marker myosin heavy chain (Figure 11). This indicates that ADC cells are capable of homing to the site of injury in a damaged heart and of differentiating into cardiac myocytes. Thus, ADC cells may be capable of replenishing cardiac myocytes that are lost following a heart attack (myocardial infarction).

To extend these findings across species, engraftment of donor derived processed lipoaspirate in a rat occlusion/reperfusion model was studied. In this experimental set-up, the main coronary artery (left anterior descending) of an immunocompetent Wistar rat was temporarily occluded using 7-0 prolene and a small piece of silastic tubing acting as a snare over the artery. After one hour, the occlusion is released and blood is allowed to reperfuse the ischemic myocardium. This model more closely represents the mechanisms of injury and repair present in the human clinical paradigm. Immediately after reperfusion, approximately 1 million (1 x 10⁶) ADC cells obtained from Rosa 26 mice were injected via an intra-ventricular route. Hearts were harvested one week following injection. As shown in Figure 12, the results demonstrate engraftment of donor derived ADC cells.

### EXAMPLE 7: Treatment of Acute Heart Damage

Acute myocardial infarct (heart attack) results in ischemic injury to the myocardium. Tissue damage can be minimized by reperfusion of the damaged tissue and by regeneration of myocardial tissue (Murry et al., 1996; Orlic et al., 2001; Orlic et al., 2003; Rajnoch et al., 2001; Strauer et al., 2002; Assmus et al., 2002). Adipo-derived cellular therapy, as disclosed herein, seeks to provide a superior source of regenerative cells relative to non-adipo-derived cellular therapies, due to for example at least one of the use of a greater number of non-cultured cells and more pure cells with attenuated morbidity associated with non-adipo-derived therapies, such as bone marrow harvesting.

A patient is suspected of having suffered from a myocardial infarction. The patient is admitted within an hour of experiencing the infarction. The patient is prescribed an adipo-derived cellular therapy. The patient's habitus is examined for a site suitable for adipose tissue collection. Harvest sites are characterized by at least one of the following: potential space(s) limited by normal anatomical structures, no major vascular or visceral structures at risk for damage, and ease of access. Virgin harvest sites are preferred, but a previous harvest site does not preclude additional adipose tissue harvest. Potential harvest sites include, but are not limited to, the following: lateral and medial thigh regions of bilateral lower extremities, anterior abdominal wall pannus, and bilateral flank regions.

The patient receives a subcutaneous injection of a tumescent fluid solution containing a combination of lidocaine, saline, and epinephrine in for example different standardized dosing regimens. Using a scalpel (e.g., an 11-blade scalpel), a small puncture wound is made in the patient's medial thigh region of his right and/or left legs in order to transverse the dermis. The blade is turned 360 degrees to complete the wound. A blunt tip cannula (e.g., 14-guage cannula) is inserted into the subcutaneous adipose tissue plane below the incision. The cannula is connected to a power assisted suction device. The cannula is moved throughout the adipose tissue plane to disrupt the connective tissue architecture. Approximately 500 cc of aspirate is obtained. After removal of the adipose tissue, hemostasis is achieved with standard surgical techniques and the wound is closed.

The lipoaspirate is processed in accordance with the methods disclosed hereinabove to obtain a unit of concentrated adipo-derived stem cells. Approximately six hours after the infarction, the patient is administered the stem cells. Based on the processing of the lipoaspirate, it is estimated that the patient receives an initial dose of stem cells in a range of between approximately 5.5 x 10⁴ stem cells and 5.5 x 10⁵ stem cells. The patient receives two supplemental dosages at 12 hour intervals after the initial administration. The stem cells are administered to the patient through a central venous catheter. To promote cellular engraftment in the target region, the flow of stem cells is controlled by a balloon located downstream of the target site and by a balloon upstream of the target site to create regions of low or minimal blood flow.

Improvements in the patient are noted within approximately six hours after the cell administration procedure. Several days after the cell administration procedure further improvement of the patient is noted evidenced by increased blood volume ejection fraction, decreased rate of heart failure, decreased infarct size, improved exercise tolerance and other quality of life measures.

### REFERENCES

Abbate,A., Biondi-Zoccai,G.G., and Baldi,A. (2002). "Pathophysiologic role of myocardial apoptosis in post-infarction left ventricular remodeling." J Cell Physiol 193, 145-53.
Aharinejad,S., Marks,S.C., Jr., Bock,P., Mason-Savas,A., MacKay,C.A., Larson,E.K., Jackson,M.E., Luftensteiner,M., and Wiesbauer,E. (1995). "CSF-1 treatment promotes angiogenesis in the metaphysics of osteopetrotic (toothless, tl) rats." Bone 16, 315-324.
Alison,M. (1998). "Liver stem cells: a two compartment system" Curr Opin Cell Biol 10, 710-5.
American-Heart-Association (2002). Heart Disease and Stroke Statistics-2003 Update. (Dallas, Texas: American Heart Association).
Arvidsson,A., Collin,T., Kirik,D., Kokaia,Z., and Lindvall,O. (2002). "Neuronal replacement from endogenous precursors in the adult brain after stroke." Nat Med 8, 963-70.
Asahara,T., Takahashi,T., Masuda,H., Kalka,C., Chen,D., Iwaguro,H., Inai,Y., Silver,M., and Isner,J.M. (1999). "VEGF contributes to postnatal neovascularization by mobilizing bone marrow-derived endothelial progenitor cells." Embo J 18, 3964-72.
Ashjian,P.H., Elbarbary,A.S., Edmonds,B., DeUgarte,D., Zhu,M., Zuk,P.A., Lorenz,H.P., Benhaim,P., and Hedrick,M.H. (2003). "In vitro differentiation of human processed lipoaspirate cells into early neural progenitors." Plast Reconstr Surg 111, 1922-31.
Asken,S. (1990). "Microliposuction and autologous fat transplantation for aesthetic enhancement of the aging face." J Dermatol Surg Oncol 16, 965-72.
Asou,Y., Rittling, S.R., Yoshitake, H., Tsuji, K., Shinomiya,K., Nifuji,A., Denhardt,D.T., and Noda,M. (2001). "Osteopontin facilitates angiogenesis, accumulation of osteoclasts, and resorption in ectopic bone." Endocrinology 142, 1325-1332.
Assady,S., Maor,G., Amit,M., Itskovitz-Eldor,J., Skorecki,K.L., and Tzukerman,M. (2001). "Insulin production by human embryonic stem cells." Diabetes 50, 1691-7.
Assmus,B., Schachinger,V., Teupe,C., Britten,M., Lehmann,R., Dobert,N., Grunwald,F., Aicher,A., Urbich,C., Martin,H., Hoelzer,D., Dimmeler,S., and Zeiher,A.M. (2002). "Transplantation of Progenitor Cells and Regeneration Enhancement in Acute Myocardial Infarction (TOPCARE-AMI)." Circulation 106, 3009-17.
Athanasopoulos,T., Fabb,S., and Dickson,G. (2000). "Gene therapy vectors based on adeno-associated virus: characteristics and applications to acquired and inherited diseases (review)." Int J Mol Med 6, 363-75.
Bagnato,A. and Spinella,F. (2003). "Emerging role of endothelin-1 in tumor angiogenesis." Trends Endocrinol. Metab 14, 44-50.
Banfi,A., Bianchi,G., Galotto,M., Cancedda,R., and Quarto,R. (2001). "Bone marrow stromal damage after chemo/radiotherapy: occurrence, consequences and possibilities of treatment." LeukLymphoma 42, 863-70.
Barker,J.N., Davies,S.M., DeFor,T., Ramsay,N.K., Weisdorf,D.J., and Wagner,J.E. (2001). "Survival after transplantation of unrelated donor umbilical cord blood is comparable to that of human leukocyte antigen-matched unrelated donor bone marrow: results of a matched-pair analysis." Blood 97, 2957-61.
Barry,F.P., Boynton,R.E., Haynesworth,S., Murphy,J.M., and Zaia,J. (1999). "The monoclonal antibody SH-2, raised against human mesenchymal stem cells, recognizes an epitope on endoglin (CD105)." Biochem Biophys Res Commun 265, 134-9.
Beecken,W.D., Kramer,W., and Jonas,D. (2000). "New molecular mediators in tumor angiogenesis." J Cell Mol Med 4, 262-269.
Berdel,W.E., Fink,U., Thiel,E., Stunkel,K., Greiner,E., Schwarzkopf,G., Reichert,A., and Rastetter,J. (1982). "Purification of human monocytes by adherence to polymeric fluorocarbon. Characterization of the monocyte-enriched cell fraction." Immunobiology 163, 511-520.
Bickenbach,J.R and Dunnwald,M. (2000). "Epidermal stem cells: characteristics and use in tissue engineering and gene therapy." Adv Dermatol 16, 159-83.
Bonner-Weir,S. and Sharma,A. (2002). "Pancreatic stem cells." J Pathol. 197, 519-526.
Botta,M., Manetti,F., and Corelli,F. (2000). "Fibroblast growth factors and their inhibitors." Curr. Pharm. Des 6,1897-1924.
Buschmann,IR., Busch,H.J., Mies,G., and Hossmann,K.A. (2003). "Therapeutic induction of arteriogenesis in hypoperfused rat brain via granulocyte-macrophage colony-stimulating factor." Circulation 108, 610-615.
Caplan,A.I. and Bruder,S.P. (2001). "Mesenchymal stem cells: building blocks for molecular medicine in the 21set century." Trends Mol Med 7, 259-64.
Caplan,A.I. and Goldberg,V.M. (1999). "Principles of tissue engineered regeneration of skeletal tissues." Clin Orthop 12-6.
Carano,R.A and Filvaroff,E.H. (2003). "Angiogenesis and bone repair." Drug Discov. Today 8, 980-989.
Carmeliet,P. (2000). "Mechanisms of angiogenesis and arteriogenesis." Nat Med 6, 389-395.
Civin,C.I., Strauss,L.C., Fackler,M.J., Trischmann,T.M., Wiley,J.M., and Loken,M.R (1990). "Positive stem cell selection--basic science." Prog Clin Biol Res 333, 387-401.
Clarke,D. and Frisen,J. (2001). "Differentiation potential of adult stem cells." Curr Opin Genet Dev 11, 575-80.
Coleman,S.R. (1995). "Long-term survival of fat transplants: controlled demonstrations." Aesthetic Plast Surg 19, 421-5.
Commons,G.W., Halperin,B., and Chang,C.C. (2001). "Large-volume liposuction: a review of 631 consecutive cases over 12 years." Plast Reconstr Surg 108, 1753-63.
Crosby,H.A. and Strain.A.J. (2001). "Adult liver stem cells: bone marrow, blood, or liver derived?" Gut 48, 153-4.
D'Ippolito,G., Schiller,P.C., Ricordi,C., Roos,B.A., and Howard,G.A. (1999). "Age-related osteogenic potential of mesenchymal stromal stem cells from human vertebral bone marrow." J Bone Miner Res 14, 1115-22.
De Ugarte,DA., Ashjian,P.H., Elbarbary,A., and Hedrick,M.H. (2003). "Future of fat as raw material for tissue regeneration." Ann Plast Surg 50, 215-9.
Donovan,D., Brown,N.J., Bishop,E.T., and Lewis,C.E. (2001). "Comparison of three in vitro human 'angiogenesis' assays with capillaries formed in vivo." Angiogenesis 4, 113-121.
Engelholm,S.A., Spang-Thomsen,M., Brunner,N., Nohr,I., and Vindelov,L.L. (1985). "Disaggregation of human solid tumors by combined mechanical and enzymatic methods." Br J Cancer 51, 93-8.
Eppley,B.L., Smith,P.G., Sadove,A.M., and Delfino,J.J. (1990). "Experimental effects of graft revascularization and consistency on cervicofacial fat transplant survival." J Oral Maxillofac Surg 48, 54-62.
Eschenhagen,T., Didie,M., Munzel,F., Schubert,P., Schneiderbanger,K., and Zimmermann,W.H. (2002). "3D engineered heart tissue for replacement therapy." Basic Res Cardiol 97 Suppl 1, I146-I152.
Falla,N., Van,V., Bierkens,J., Borremans,B., Schoeters,G., and Van Gorp,U. (1993). "Characterization of a 5-fluorouracil-enriched osteoprogenitor population of the murine bone marrow." Blood 82, 3580-91.
Folkman,J. (1995). "Angiogenesis in cancer, vascular, rheumatoid and other disease." Nat Med 1, 27-31.
Formanek,M., Temmel,A., Knerer,B., Willheim,M., Millesi,W., and Kornfehl,J. (1998). "Magnetic cell separation for purification of human oral keratinocytes: an effective method for functional studies without prior cell subcultivation." Eur Arch. Otorhinolaryngol. 255, 211-215.
Fukuda,K. (2001). "Development of regenerative cardiomyocytes from mesenchymal stem cells for cardiovascular tissue engineering." Artif Organs 25, 187-93.
Gaustad,K.G., Boquest,A.C., Anderson,B.E., Gerdes,A.M., and Collas,P. (2004). "Differentiation of human adipose tissue stem cells using extracts of rat cardiomyocytes." Biochem. Biophys. Res Commun. 314, 420-427.
Geisellhart,A., Neu,S., BuchholzF., Lang,P., Niethammer,D., and Handgretinger,R. (1996). "Positive selection of CD56+ lymphocytes by magnetic cell sorting." Nat Immun. 15, 227-233.
Gimble,J.M., Morgan,C., Kelly,K., Wu,X., Dandapani,V., Wang,C.S., and Rosen,V. (1995). "Bone morphogenetic proteins inhibit adipocyte differentiation by bone marrow stromal cells." J Cell Biochem 58, 393-402.
Graepler,F., Lauer,U., and Gregor,M. (1998). "Magnetic cell sorting for parietal cell purification using a new monoclonal antibody without influence on cell function." J Biochem. Biophys. Methods 36, 143-155.
Greenberg,A.W. and Hammer,D.A. (2001). "Cell separation mediated by differential rolling adhesion." Biotechnol Bioeng 73, 111-24.
Gryn,J., Shadduck,R.K., Lister,J., Zeigler,Z.R., and Raymond,J.M. (2002). "Factors affecting purification of CD34(+) peripheral blood stem cells using the Baxter Isolex 300i." J Hematother Stem Cell Res 11, 719-30.
Hagege,A.A., Camion,C., Menasche,P., Vilquin,J.T., Duboc,D., Marolleau,J.P., Desnos,M., and Bruneval,P. (2003). "Viability and differentiation of autologous skeletal myoblast grafts in ischaemic cardiomyopathy." Lancet 361, 491-2.
Hanada,K., Dennis,J.E., and Caplan,A.I. (1997). "Stimulatory effects of basic fibroblast growth factor and bone morphogenetic protein-2 on osteogenic differentiation of rat bone marrow-derived mesenchymal stem cells." J Bone Miner Res 12, 1606-14.
Hemstreet,G.P.3., Enoch,P.G., and Pretlow,T.G.2. (1980). "Tissue disaggregation of human renal cell carcinoma with further isopyknic and isokinetic gradient purification.". Cancer Res 40, 1043-9.
Horwitz,E.M., Prockop,D.J., Gordon,P.L., Koo,W.W., Fitzpatrick,L.A., Neel,M.D., McCarville,M.E., Orchard,P.J., Pyeritz,RE., and Brenner,M.K. (2001). "Clinical responses to bone marrow transplantation in children with severe osteogenesis imperfecta." Blood 97, 1227-31.
Ito,Y. and Shinomiya,K. (2001). "A new continuous-flow cell separation method based on cell density: principle, apparatus, and preliminary application to separation of human buffy coat." J Clin Apheresis. 16, 186-191.
Jacobson,L., Kahan,B., Djamali,A., Thomson,J., and Odorico,J.S. (2001). "Differentiation of endoderm derivatives, pancreas and intestine, from rhesus embryonic stem cells." Transplant Proc 33, 674.
Jaiswal,R.K., Jaiswal,N., Bruder,S.P., Mbalaviele,G., Marshak,D.R, and Pittenger,M.F. (2000). "Adult human mesenchymal stem cell differentiation to the osteogenic or adipogenic lineage is regulated by mitogen-activated protein kinase." J Biol Chem 275, 9645-52.
Jiang,Y., Jahagirdar.B.N., Reinhardt,R.L., Schwartz.R.E., Keene,C.D., Ortiz-Gonzalez,X.R., Reyes,M., Lenvik,T., Lund,T., Blackstad,M., Du,J., Aldrich,S., Lisberg,A., Low,W.C., "Largaespada,D.A., and Verfaillie,C.M. (2002a). Pluripotency of mesenchymal stem cells derived from adult marrow." Nature 418, 41-9.
Jiang,Y., Vaessen,B., Lenvik,T., Blackstad,M., Reyes,M., and Verfaillie,C.M. (2002b). "Multipotent progenitor cells can be isolated from postnatal murine bone marrow, muscle, and brain." Exp Hematol 30, 896-904.
Joyner,C.J., Triffitt,J., Puddle,B., and Athanasou,N.A. (1999). "Development of a monoclonal antibody to the aP2 protein to identify adipocyte precursors in tumours of adipose differentiation." Pathol. Res Pract. 195, 461-466.
Katz,A.J., Hedrick,M.H., Llull,R., and Futrell,J.W. (2001a). "A novel device for the simple and efficient refinement of liposuctioned tissue." Plast Reconstr Surg 107, 595-7.
Katz,B.E., Bruck,M.C., and Coleman,W.P.3. (2001b). "The benefits of powered liposuction versus traditional liposuction: a paired comparison analysis." Dermatol Surg 27, 863-7.
Kaushal,S., Amiel,G.E., Guleserian,K.J., Shapira,O.M., Perry,T., Sutherland,F.W., Rabkin,E., Moran,A.M., Schoen,F.J., Atala,A., Soker.S.. Bischoff,J., and Mayer,J.F., Jr. (2001). "Functional small-diameter neovessels created using endothelial progenitor cells expanded ex vivo." Nat Med 7, 1035-40.
Kawamoto,A., Gwon,H.C., Iwaguro,H., Yamaguchi,J.I., Uchida,S., Masuda,H., Silver,M., Ma,H., Kearney,M., Isner,J.M., and Asahara,T. (2001). "Therapeutic potential of ex vivo expanded endothelial progenitor cells for myocardial ischemia." Circulation 103, 634-7.
Kawamoto,A., Tkebuchava,T., Yamaguchi,J., Nishimura,H., Yoon,Y.S., Milliken,C., Uchida,S., Masuo,O., Iwaguro,H., Ma,H., Hanley,A., Silver,M., Kearney,M., Losordo,D.W., Isner,J.M., and Asahara,T. (2003). "Intramyocardial transplantation of autologous endothelial progenitor cells for therapeutic neovascularization of myocardial ischemia." Circulation 107, 461-8.
Kobari,L., Giarratana,M.C., Pflumio,F., Izac,B., Coulombel,L., and Douay,L. (2001). "CD133+ cell selection is an alternative to CD34+ cell selection for ex vivo expansion of hematopoietic stem cells." J Hematother Stem Cell Res 10, 273-281.
Lamouille,S., Mallet,C., Feige,J.J., and Bailly,S. (2002). "Activin receptor-like kinase 1 is implicated in the maturation phase of angiogenesis." Blood 100, 4495-4501.
Lasch,J., Kullertz,G., and Opalka,J.R. (2000). "Separation of erythrocytes into age-related fractions by density or size? Counterflow centrifugation." Clin Chem Lab Med 38, 629-632.
Lehner,M. and Holter,W. (2002). "Endotoxin-free purification of monocytes for dendritic cell generation via discontinuous density gradient centrifugation based on diluted Ficoll-Paque Plus." Int Arch. Allergy Immunol. 128, 73-76.
Lennon,D.P., Haynesworth,S.E., Young,RG., Dennis,J.E., and Caplan,A.I. (1995). "A chemically defined medium supports in vitro proliferation and maintains the osteochondral potential of rat marrow-derived mesenchymal stem cells." Exp Cell Res 219, 211-22.
Lieberman,J.R., Le,L.Q., Wu,L., Finerman,G.A., Berk,A., Witte,O.N., and Stevenson,S. (1998). "Regional gene therapy with a BMP-2-producing murine stromal cell line induces heterotopic and orthotopic bone formation in rodents." J Orthop Res 16, 330-9.
Lind,M. (1998). "Growth factor stimulation of bone healing. Effects on osteoblasts, osteomies, and implants fixation." Acta Orthop Scand Suppl 283, 2-37.
Luskey,B.D., Lim,B., Apperley,J.F., Orkin,S.H., and Williams,D.A. (1990). "Gene transfer into murine hematopoietic stem cells and bone marrow stromal cells." Ann N YAcad Sci 612, 398-406.
Luttun,A., Tjwa,M., Moons,L., Wu,Y., Angelillo-Scherrer,A., Liao,F., Nagy,J.A., Hooper,A., Priller,J., De Klerck,B., Compernolle,V., Daci,E., Bohlen,P., Dewerchin,M., Herbert,J.M., Fava,R, Matthys,P., Carmeliet,G., Collen,D., Dvorak,H.F., Hicklin,D.J., and Carmeliet,P. (2002). "Revascularization of ischemic tissues by P1GF treatment, and inhibition of tumor angiogenesis, arthritis and atherosclerosis by anti-Flt1." Nat Med 8, 831-40.
Mainwaring,G. and Rowley,A.F. (1985). "Separation of leucocytes in the dogfish (Scyliorhinus canicula) using density gradient centrifugation and differential adhesion to glass coverslips." Cell Tissue Res 241, 283-90.
Majumdar,M.K., Thiede,M.A., Mosca,J.D., Moorman,M., and Gerson,S.L. (1998). "Phenotypic and functional comparison of cultures of marrow-derived mesenchymal stem cells (MSCs) and stromal cells." J Cell Physiol 176, 57-66.
Marchlinski,F.E., Falcone,R., Iozzo,R.V., Reichek,N., Vassallo,J.A., and Eysmann,S.B. (1987). "Experimental myocardial cryoinjury: local electromechanical changes, arrhythmogenicity, and methods for determining depth of injury." Pacing Clin Electrophysiol 10, 886-901.
Mills,J.D., Fischer,D., and Villanueva,F.S. (2000). "Coronary collateral development during chronic ischemia: serial assessment using harmonic myocardial contrast echocardiography." J Am Coll Cardiol 36, 618-624*.*
Mints,M., Blomgren,B., Falconer,C., and Palmblad,J. (2002). "Expression of the vascular endothelial growth factor (VEGF) family in human endometrial blood vessels." Scand. J Clin Lab Invest 62, 167-175*.*
Mizuno,H., Zuk,P.A., Zhu,M., Lorenz,H.P., Benhaim,P., and Hedrick,M.H. (2002). "Myogenic differentiation by human processed lipoaspirate cells." Plast Reconstr Surg 109, 199-209.
Mohr,M., Dalmis,F., Hilgenfeld,E., Oelmann,E., Zuhlsdorf,M., Kratz-Albers,K., Nolte,A., Schmitmann,C., Onaldi-Mohr,D., Cassens,U., Serve,H., Sibrowski,W., Kienast,J., and Berdel,W.E. (2001). "Simultaneous immunomagnetic CD34+ cell selection and B-cell depletion in peripheral blood progenitor cell samples of patients suffering from B-cell non-Hodgkin's lymphoma." Clin Cancer Res 7, 51-57*.*
Monteiro,P., Antunes,A., Goncalves,L.M., and Providencia,L.A. (2003). "Long-term clinical impact of coronary-collateral vessels after acute myocardial infarction." Rev. Port. Cardiol 22, 1051-1061.
Morizono,K., De Ugarte,D.A., Zhu,M., Zuk,P., Elbarbary,A., Ashjian,P., Benhaim,P., Chen,I.S., and Hedrick,M.H. (2003). "Multilineage cells from adipose tissue as gene delivery vehicles." Hum. Gene Ther. 14, 59-66.
Mosca,J.D., Hendricks,J.K, Buyaner,D., Davis-Sproul,J., Chuang,L.C., Majumdar,M.K., Chopra,R., Barry,F., Murphy,M., Thiede,M.A., Junker,U., Rigg,R.J., Forestell,S.P., Bohnlein,E., Storb.R., and Sandmaier,B.M. (2000). "Mesenchymal stem cells as vehicles for gene delivery." Clin Orthop 71-90.
Muramatsu,T., Nakamura,A., and Park,H.M. (1998). "In vivo electroporation: a powerful and convenient means of nonviral gene transfer to tissues of living animals (Review)." Int J Mol Med 1, 55-62.
Murry,C.E., Wiseman,R.W., Schwartz,S.M., and Hauschka,S.D. (1996). "Skeletal myoblast transplantation for repair of myocardial necrosis." J Clin Invest 98, 2512-23.
Muschler,G.F., Nitto,H., Boehm,C.A., and Easley,K.A. (2001). "Age- and gender-related changes in the cellularity of human bone marrow and the prevalence of osteoblastic progenitors." J Orthop Res 19, 117-25.
Nagy,J.A., Dvorak,A.M., and Dvorak,H.F. (2003). "VEGF-A(164/165) and P1GF: roles in angiogenesis and arteriogenesis." Trends Cardiovasc Med 13, 169-175.
Nerem,R.M. and Ensley,A.E. (2004). "The tissue engineering of blood vessels and the heart." Am j Transplant 4 Suppl 6, 36-42.
Nishimori,M., Yamada,Y., Hoshi,K., Akiyama,Y., Hoshi,Y., Morishima,Y., Tsuchida,M., Fukuhara,S., and Kodera,Y. (2002). "Health-related quality of life of unrelated bone marrow donors in Japan." Blood 99,1995-2001.
Odorico,J.S., Kaufman,D.S., and Thomson,J.A. (2001). "Multilineage differentiation from human embryonic stem cell lines." Stem Cells 19, 193-204.
Ohgushi,H. and Caplan,A.I. (1999). "Stem cell technology and bioceramics: from cell to gene engineering." J Biomed Mater Res 48, 913-27.
Orlic,D., Kajstura,J., Chimenti,S., Bodine,D.M., Leri,A., and Anversa,P. (2001). "Transplanted adult bone marrow cells repair myocardial infarcts in mice." Ann N Y Acad Sci 938, 221-9.
Orlic,D., Kajstura,J., Chimenti,S., Bodine,D.M., Leri,A., and Anversa,P. (2003). "Bone marrow stem cells regenerate infarcted myocardium." Pediatr. Transplant. 7 Suppl 3, 86-88.
Pera,M.F., Reubinoff,B., and Trounson,A. (2000). "Human embryonic stem cells." J Cell Sci 113 (Pt 1), 5-10.
Pipp,F., Heil,M., Issbrucker,K., Ziegelhoeffer,T., Martin,S., van den,H.J., Weich,H., Fernandez,B., Golomb,G., Carmeliet,P., Schaper,W., and Clauss,M. (2003). "VEGFR-1-selective VEGF homologue P1GF is arteriogenic: evidence for a monocyte-mediated mechanism." Circ. Res 92, 378-385.
Pittenger,M.F., Mackay,A.M., Beck,S.C., Jaiswal,RK., Douglas,R., Mosca,J.D., Moorman,M.A., Simonetti,D.W., Craig,S., and Marshak,D.R. (1999). "Multilineage potential of adult human mesenchymal stem cells." Science 284, 143-7.
Prince,H.M., Bashford,J., Wall,D., Rischin,D., Parker,N., Toner,G.C., Seymour,J.F., Blakey,D., Haylock,D., Simmons,P., Francis,P., Wolf,M., Januszewicz,E.H., Richardson,G., Scarlett,J., and Briggs,P. (2002). "Isolex 300i CD34-selected cells to support multiple cycles of high-dose therapy." Cytotherapy 4, 137-45.
Qian,X., Jin,L., and Lloyd,R.V. (1998). "Percoll Density Gradient-Enriched Populations of Rat Pituitary Cells: Interleukin 6 Secretion, Proliferative Activity, and Nitric Oxide Synthase Expression." Endocr. Pathol. 9, 339-346.
Quirici,N., Soligo,D., Bossolasco,P., Servida,F., Lumini,C., and Deliliers,G.L. (2002). "Isolation of bone marrow mesenchymal stem cells by anti-nerve growth factor receptor antibodies." Exp Hematol 30, 783-91.
Rajnoch,C., Chachques,J.C., Berrebi,A., Bruneval,P., Benoit,M.O., and Carpentier,A. (2001). "Cellular therapy reverses myocardial dysfunction." J Thorac Cardiovasc Surg 121, 871-8.
Rangappa,S., Fen,C., Lee,E.H., Bongso,A., and Wei,E.S. (2003). "Transformation of adult mesenchymal stem cells isolated from the fatty tissue into cardiomyocytes." Ann Thorac. Surg 75, 775-779.
Remme,W.J. (2000). "Overview of the relationship between ischemia and congestive heart failure." Clin Cardiol 23, 4-8.
Reyes,M., Lund,T., Lenvik,T., Aguiar,D., Koodie,L., and Verfaillie,C.M. (2001). "Purification and ex vivo expansion of postnatal human marrow mesodermal progenitor cells." Blood 98, 2615-2625.
Ribatti,D., Vacca,A., Roccaro,A.M., Crivellato,E., and Presta,M. (2003). "Erythropoietin as an angiogenic factor." Eur J Clin Invest 33, 891-896.
Russell,S.W., Doe,W.F., Hoskins,R.G., and Cochrane,C.G. (1976). "Inflammatory cells in solid murine neoplasms. I. Tumor disaggregation and identification of constituent inflammatory cells." Int J Cancer 18, 322-30.
Scholz,D., Cai,W.J., and Schaper,W. (2001). "Arteriogenesis, a new concept of vascular adaptation in occlusive disease." Angiogenesis. 4, 247-257.
Scholz,D., Elsaesser,H., Sauer,A., Friedrich,C., Luttun,A., Carmeliet,P., and Schaper,W. (2003). "Bone marrow transplantation abolishes inhibition of arteriogenesis in placenta growth factor (P1GF) -/- mice." J Mol Cell Cardiol 35, 177-184.
Scholz,D., Ziegelhoeffer,T., Helisch,A., Wagner,S., Friedrich,C., Podzuweit,T., and Schaper,W. (2002). "Contribution of arteriogenesis and angiogenesis to postocclusive hindlimb perfusion in mice." J Mol Cell Cardiol 34, 775-787.
Schwartz,R.E., Reyes,M., Koodie,L., Jiang,Y., Blackstad,M., Lund,T., Lenvik,T., Johnson.S., Hu,W.S., and Verfaillie,C.M. (2002). "Multipotent adult progenitor cells from bone marrow differentiate into functional hepatocyte-like cells." J Clin Invest 109, 1291-302.
Schweitzer,C.M., van, der, Schoot, Ce, Drager,A.M., van, der, Valk, P, Zevenbergen,A., Hooibrink,B., Westra,A.H., and Langenhuijsen,M.M. (1995). "Isolation and culture of human bone marrow endothelial cells." Exp Hematol 23, 41-8.
Sekiya,I., Larson,B.L., Smith,J.R., Pochampally,R., Cui,J.G., and Prockop,D.J. (2002). "Expansion of human adult stem cells from bone marrow stroma: conditions that maximize the yields of early progenitors and evaluate their quality." Stem Cells 20, 530-541.
Sergeant,P., Blackstone,E., and Meyns,B. (1997). "Early and late outcome after CABG in patients with evolving myocardial infarction." Eur J Cardiothorac. Surg 11, 848-856.
Shigematsu,S., Yamauchi,K., Nakajima,K., Iijima,S., Aizawa,T., and Hashizume,K. (1999). "IGF-1 regulates migration and angiogenesis of human endothelial cells." Endocr. J 46 Suppl, S59-S62.
Smith,J.W. (1997). "Apheresis techniques and cellular immunomodulation." Ther. Apher. 1, 203-206.
Smith,R.J. and Sweetenham,J.W. (1995). "A mononuclear cell dose of 3 x 10(8)/kg predicts early multilineage recovery in patients with malignant lymphoma treated with carmustine, etoposide, Ara-C and melphalan (BEAM) and peripheral blood progenitor cell transplantation." Exp Hemato1 23, 1581-8*.*
Smits,G., Holzgreve,W., and Hahn,S. (2000). "An examination of different Percoll density gradients and magnetic activated cell sorting (MACS) for the enrichment of fetal erythroblasts from maternal blood." Arch. Gynecol. Obstet. 263, 160-163.
Sodian,R., Lemke,T., Fritsche,C., Hoerstrup,S.P., Fu,P., Potapov,E.V., Hausmann,H., and Hetzer,R. (2002). "Tissue-engineering bioreactors: a new combined cell-seeding and perfusion system for vascular tissue engineering." Tissue Eng 8, 863-870.
Soli,M., Blanco,L., Riggert,J., Martinez-Clavel,A., Lucas,C., Lunghi,M., Belloni,M., Wolf,C., van Waeg,G., and Antoon,M. (2001). "A multicentre evaluation of a new filtration protocol for leucocyte depletion ofhigh-haematocrit red blood cells collected by an automated blood collection system." Vox Sang. 81, 108-112.
Stamm,C., Westphal,B., Kleine,H.D., Petzsch,M., Kittner,C., Klinge,H., Schumichen,C., Nienaber,C.A., Freund,M., and Steinhoff,G. (2003). "Autologous bone-marrow stem-cell transplantation for myocardial regeneration." Lancet 4, 45-46.
Strauer,B.E., Brehm,M., Zeus,T., Kostering,M., Hernandez,A., Sorg,R.V., Kogler,G., and Wernet,P. (2002). "Repair of infarcted myocardium by autologous intracoronary mononuclear bone marrow cell transplantation in humans." Circulation 106, 1913-8.
Sundberg,C., Kowanetz,M., Brown,L.F., Detmar,M., and Dvorak,H.F. (2002). "Stable expression of angiopoietin-1 and other markers by cultured pericytes: phenotypic similarities to a subpopulation of cells in maturing vessels during later stages of angiogenesis in vivo." Lab Invest 82, 387-401.
Toma,J.G., Akhavan,M., Fernandes,K.J., Barnabe-Heider,F., Sadikot,A., Kaplan,D.R., and Miller,F.D. (2001). "Isolation of multipotent adult stem cells from the dermis of mammalian skin." Nat Cell Biol 3, 778-84.
Twentyman,P.R. and Yuhas,J.M. (1980). "Use of bacterial neutral protease for disaggregation of mouse tumours and multicellular tumor spheroids." Cancer Lett 9, 225-8.
Van,M., V, Meyer,E., Dosogne,H., and Burvenich,C. (2001). "Separation of bovine bone marrow into maturation-related myeloid cell fractions." Vet. Immunol. Immunopathol. 83, 11-17.
Walther,W. and Stein,U. (2000). "Viral vectors for gene transfer: a review of their use in the treatment of human diseases." Drugs 60, 249-71.
Wang,L., Zeng,H., Wang,P., Soker,S., and Mukhopadhyay,D. (2003). "Neuropilin-1-mediated vascular permeability factor/vascular endothelial growth factor-dependent endothelial cell migration." J Biol Chem 278, 48848-48860.
Watts,M.J., Somervaille,T.C., Ings,S.J., Ahmed,F., Khwaja,A., Yong,K., and Linch,D.C. (2002). "Variable product purity and functional capacity after CD34 selection: a direct comparison of the CliniMACS (v2.1) and Isolex 300i (v2.5) clinical scale devices." Br J Haematol 118, 117-23.
Williams,S.K., McKenney,S., and Jarrell,B.E. (1995). "Collagenase lot selection and purification for adipose tissue digestion." Cell Transplant 4, 281-9.
Worster,A.A., Brower-Toland,B.D., Fortier,L.A., Bent,S.J., Williams,J., and Nixon,A.J. (2001). "Chondrocytic differentiation of mesenchymal stem cells sequentially exposed to transforming growth factor-betal in monolayer and insulin- like growth factor-I in a three-dimensional matrix." J Orthop Res 19, 738-49.
Xiong,B., Gong,L.L., Zhang,F., Hu,M.B., and Yuan,H.Y. (2002). "TGF beta1 expression and angiogenesis in colorectal cancer tissue." World J Gastroenterol. 8, 496-498.
Ye,Q., Zund,G., Benedikt,P., Jockenhoevel,S., Hoerstrup,S.P., Sakyama,S., Hubbell,J.A., and Turina,M. (2000). "Fibrin gel as a three dimensional matrix in cardiovascular tissue engineering." Eur J Cardiothorac Surg 17, 587-91.
Young,H.E., Steele,T.A., Bray,R.A., Hudson,J., Floyd,J.A., Hawkins,K., Thomas,K., Austin,T., Edwards,C., Cuzzourt,J., Duenzl,M., Lucas,P.A., and Black,A.C., Jr. (2001). "Human reserve pluripotent mesenchymal stem cells are present in the connective tissues of skeletal muscle and dermis derived from fetal, adult, and geriatric donors." Anat Rec 264, 51-62.
Zimmermann,W.H., Didie,M., Wasmeier,G.H., Nixdorff,U., Hess,A., Melnychenko,I, Boy,O., Neuhuber,W.L., Weyand,M., and Eschenhagen,T. (2002). "Cardiac grafting of engineered heart tissue in syngenic rats." Circulation 106, I151-I157.
Zimmermann,W.H., Melnychenko,I., and Eschenhagen,T. (2004). "Engineered heart tissue for regeneration of diseased hearts." Biomaterials 25, 1639-1647.
Zuk,P.A., Zhu,M., Ashjian,P., De Ugarte,D.A., Huang,J.I., Mizuno,H., Alfonso,Z.C., Fraser,J.K., Benhaim,P., and Hedrick,M.H. (2002). "Human adipose tissue is a source of multipotent stem cells.' Mol Biol Cell 13, 4279-95.
Zuk,P.A., Zhu,M., Mizuno,H., Huang,J., Futrell,J.W., Katz,A.J., Benhaim,P., Lorenz,H.P., and Hedrick,M.H. (2001). "Multilineage cells from human adipose tissue: implications for cell-based therapies." Tissue Eng 7, 211-28.

Any feature or combination of features described herein are included within the scope of the present invention provided that the features included in any such combination are not mutually inconsistent as will be apparent from the context, this specification, and the knowledge of one of ordinary skill in the art. For purposes of summarizing the present invention, certain aspects, advantages and novel features of the present invention have been described herein. Of course, it is to be understood that not necessarily all such aspects, advantages or features will be embodied in any particular embodiment of the present invention. Additional advantages and aspects of the present invention are apparent in the following detailed description and claims.

The above-described embodiments have been provided by way of example, and the present invention is not limited to these examples. Multiple variations and modification to the disclosed embodiments will occur, to the extent not mutually exclusive, to those skilled in the art upon consideration of the foregoing description. Additionally, other combinations, omissions, substitutions and modifications will be apparent to the skilled artisan in view of the disclosure herein. Accordingly, the present invention is not intended to be limited by the disclosed embodiments, but is to be defined by reference to the appended claims.

### EQUIVALENTS

Those skilled in the art will recognize , or be able to ascertain using no more than routine experimentation, many equivalents to the specific embodiments of the invention described herein. Such equivalents are intended to be encompassed by the following claims.

## Claims

1. Use of a composition comprising a concentrated population of adipose-derived cells (ADC) in the manufacture of a medicament for restoring blood flow in a subject that is evaluated as having an ischemia, wherein said concentrated population of adipose-derived cells (ADC) comprises adipose derived progenitor cells and adipose stem cells obtained by processing adipose tissue from said patient in a system, which is configured to maintain a closed sterile fluid/tissue pathway, said system comprising a collection chamber connected to a mixing/processing chamber.

2. Use in accordance with claim 1, wherein said concentrated cell population comprises at least 500 endothelial progenitor cells/million adipose derived cells.

3. Use in accordance with claim 1, wherein said ischemic condition comprises congestive heart failure.

4. Use in accordance with claim 1, wherein said ischemic condition is myocardial infarction.

5. Use in accordance with claim 1, wherein said subject is diabetic.

6. Use in accordance with claim 1, wherein said subject is obese.

7. Use in accordance with claim 1, wherein said composition comprises a bolus of said adipose derived cells.

8. Use in accordance with claim 1, wherein said composition provides between 55,000 and 550,000 adipose-derived stern cells.

9. Use in accordance with claim 1, wherein said composition further comprises one or more angiogenic factors.

10. Use in accordance with claim 1, wherein said cell population is **characterized** as having expression of vascular endothelial growth factor (VEGF) when cultured.

11. Use in accordance with claim 1, wherein said cell population is **characterized** as having expression of placental growth factor (PLGF) when cultured.

12. Use in accordance with claim 1, wherein said composition further comprises one or more arteriogenic factors.

13. Use in accordance with claim 1, wherein said composition further comprises one or more immunosuppressive drugs.

14. Use in accordance with claim 1, wherein said adipose derived cells are not grown in culture.

15. Use in accordance with claim 1, wherein said adipose derived cells are grown in cell culture.

16. Use in accordance with claim 15, wherein said adipose derived cells are grown in culture conditions that promote differentiation towards a myocytic phenotype.

17. Use in accordance with claim 16 wherein said myocytic phenotype is a cardiac myocytic phenotype.

18. Use in accordance with claim 16, wherein said myocytic phenotype is a skeletal myocytic phenotype.

19. Use in accordance with claim 16, wherein said myocytic phenotype is a vascular smooth muscle myocytic phenotype.

20. Use in accordance with claim 15, wherein said adipose derived cells are grown in culture conditions that promote differentiation towards an endothelial phenotype.

21. Use in accordance with claim 15, wherein said cell culture is performed on a scaffold material to generate a two or three dimensional construct that can be placed on or within the heart.

22. Use in accordance with claim 21, wherein said scaffold material is resorbable in vivo.

23. Use in accordance with claim 1, wherein the adipose-derived endothelial progenitor cells are modified by gene transfer.

24. Use in accordance with claim 23, wherein said modification alters angiogenesis.

25. Use in accordance with claim 23, wherein said modification alters arteriogenesis.

26. Use in accordance with claim 23, wherein said modification alters apoptosis.

27. Use in accordance with claim 26, wherein said modified adipose-derived endothelial progenitor cells can differentiate into cardiac myocytes with altered apoptosis.

28. Use in accordance with claim 23, wherein said modification alters the homing properties of said adipose derived cells.

## Patentansprüche

1. Verwendung einer Zusammensetzung, umfassend eine konzentrierte Population aus Fettgewebe abgeleiteter Zellen (ADC) zur Herstellung eines Arzneimittels zur Wiederherstellung des Blutflusses bei einem Patienten, bei dem eine Ischämie diagnostiziert wurde, wobei die konzentrierte Population aus Fettgewebe abgeleiteter Zellen (ADC) aus Fettgewebe abgeleitete Progenitorzellen und aus Fettgewebe abgeleitete Stammzellen umfasst, die durch Aufarbeiten von Fettgewebe des Patienten in einem System erhalten werden, das zur Aufrechterhaltung eines geschlossenen sterilen Flüssigkeits-/Gewebe-Wegs konfiguriert ist, wobei das System eine Sammelkammer umfasst, die mit einer Misch-/Aufarbeitungskammer verbunden ist.

2. Verwendung nach Anspruch 1, wobei die konzentrierte Zellpopulation mindestens 500 endotheliale Progenitorzellen/Million aus Fettgewebe abgeleiteter Zellen umfasst.

3. Verwendung nach Anspruch 1, wobei der ischämische Zustand eine dekompensierte Herzinsuffizienz umfasst.

4. Verwendung nach Anspruch 1, wobei der ischämische Zustand einen Myokardinfarkt umfasst.

5. Verwendung nach Anspruch 1, wobei der Patient Diabetiker ist.

6. Verwendung nach Anspruch 1, wobei der Patient adipös ist.

7. Verwendung nach Anspruch 1, wobei die Zusammensetzung eine Bolusgabe der aus Fettgewebe abgeleiteten Zellen umfasst.

8. Verwendung nach Anspruch 1, wobei die Zusammensetzung zwischen 55.000 und 550.000 aus Fettgewebe abgeleiteten Stammzellen bereitstellt.

9. Verwendung nach Anspruch 1, wobei die Zusammensetzung weiterhin einen oder mehrere angiogene Faktoren umfasst.

10. Verwendung nach Anspruch 1, wobei die Zellpopulation derart charakterisiert ist, dass sie bei der Züchtung den vaskulären endothelialen Wachstumsfaktor (VEGF) exprimiert.

11. Verwendung nach Anspruch 1, wobei die Zellpopulation derart charakterisiert ist, dass sie bei der Züchtung den Plazentawachstumsfaktor (PLGF) exprimiert.

12. Verwendung nach Anspruch 1, wobei die Zusammensetzung weiterhin einen oder mehrere arteriogene Faktoren umfasst.

13. Verwendung nach Anspruch 1, wobei die Zusammensetzung weiterhin einen oder mehrere immunsuppressive Wirkstoffe umfasst.

14. Verwendung nach Anspruch 1, wobei die aus Fettgewebe abgeleiteten Zellen nicht in Kulturmedium gezüchtet werden.

15. Verwendung nach Anspruch 1, wobei die aus Fettgewebe abgeleiteten Zellen in einer Zellkultur gezüchtet werden.

16. Verwendung nach Anspruch 15, wobei die aus Fettgewebe abgeleiteten Zellen unter Kulturbedingungen gezüchtet werden, die eine Differenzierung zu einem myozytischen Phänotyp fördern.

17. Verwendung nach Anspruch 16, wobei der myozytische Phänotyp ein kardialer myozytischer Phänotyp ist.

18. Verwendung nach Anspruch 16, wobei der myozytische Phänotyp ein skelettaler myozytischer Phänotyp ist.

19. Verwendung nach Anspruch 16, wobei der myozytische Phänotyp ein myozytischer Phänotyp der vaskulären glatten Muskulatur ist.

20. Verwendung nach Anspruch 15, wobei die aus Fettgewebe abgeleiteten Zellen unter Kulturbedingungen gezüchtet werden, die eine Differenzierung zu einem endothelialen Phänotyp fördern.

21. Verwendung nach Anspruch 15, wobei die Zellkultivierung auf einem Gerüstmaterial durchgeführt wird, um zwei- oder dreidimensionale Konstrukte zu generieren, die auf oder in dem Herzen angebracht werden können.

22. Verwendung nach Anspruch 21, wobei das Gerüstmaterial *in vivo* resorbierbar ist.

23. Verwendung nach Anspruch 21, wobei die aus Fettgewebe abgeleiteten endothelialen Progenitorzellen durch Gentransfer modifiziert sind.

24. Verwendung nach Anspruch 23, wobei die Modifikation die Angiogenese verändert.

25. Verwendung nach Anspruch 23, wobei die Modifikation die Arteriogenese verändert.

26. Verwendung nach Anspruch 23, wobei die Modifikation die Apoptose verändert.

27. Verwendung nach Anspruch 26, wobei die modifizierten aus Fettgewebe abgeleiteten endothelialen Progenitorzellen in kardiale Myozyten mit veränderter Apoptose differenziert werden können.

28. Verwendung nach Anspruch 23, wobei die Modifikation das Wanderungsmuster der aus Fettgewebe abgeleiteten Zellen ändert.

## Revendications

1. Utilisation d'une composition comprenant une population concentrée de cellules d'origine adipeuse (ADC) dans la fabrication d'un médicament destiné à rétablir le flux sanguin chez un sujet évalué comme ischémique, dans laquelle ladite population concentrée de cellules d'origine adipeuse (ADC) comprend des cellules progénitrices d'origine adipeuse et des cellules souches adipeuses obtenues par traitement de tissu adipeux provenant dudit sujet dans un système configuré pour maintenir un circuit tissu/fluide stérile fermé, ledit système comprenant une chambre de collecte reliée à une chambre de traitement/mélange.

2. Utilisation selon la revendication 1, dans laquelle ladite population concentrée de cellules comprend au moins 500 cellules progénitrices endothéliales/million de cellules d'origine adipeuse.

3. Utilisation selon la revendication 1, dans laquelle ladite maladie ischémique comprend une insuffisance cardiaque congestive.

4. Utilisation selon la revendication 1, dans laquelle ladite maladie ischémique est un infarctus du myocarde.

5. Utilisation selon la revendication 1, dans laquelle ledit sujet est diabétique.

6. Utilisation selon la revendication 1, dans laquelle ledit sujet est obèse.

7. Utilisation selon la revendication 1, dans laquelle ladite composition comprend un bolus desdites cellules d'origine adipeuse.

8. Utilisation selon la revendication 1, dans laquelle ladite composition apporte entre 55 000 et 550 000 cellules souches d'origine adipeuse.

9. Utilisation selon la revendication 1, dans laquelle ladite composition comprend également un ou plusieurs facteurs angiogéniques.

10. Utilisation selon la revendication 1, dans laquelle ladite population de cellules est **caractérisée par** l'expression de facteur de croissance endothélial vasculaire (VEGF) lorsqu'elles sont mises en culture.

11. Utilisation selon la revendication 1, dans laquelle ladite population de cellules est **caractérisée par** l'expression de facteur de croissance placentaire (PLGF) lorsqu'elles sont mises en culture.

12. Utilisation selon la revendication 1, dans laquelle ladite composition comprend également un ou plusieurs facteurs artériogéniques.

13. Utilisation selon la revendication 1, dans laquelle ladite composition comprend également un ou plusieurs médicaments immunodépresseurs.

14. Utilisation selon la revendication 1, dans laquelle lesdites cellules d'origine adipeuse ne sont pas élevées en culture.

15. Utilisation selon la revendication 1, dans laquelle lesdites cellules d'origine adipeuse sont élevées en culture cellulaire.

16. Utilisation selon la revendication 15, dans laquelle lesdites cellules d'origine adipeuse sont élevées dans des conditions de culture qui favorisent une différenciation vers un phénotype myocytaire.

17. Utilisation selon la revendication 16, dans laquelle ledit phénotype myocytaire est un phénotype myocytaire cardiaque.

18. Utilisation selon la revendication 16, dans laquelle ledit phénotype myocytaire est un phénotype myocytaire squelettique.

19. Utilisation selon la revendication 16, dans laquelle ledit phénotype myocytaire est un phénotype myocytaire de muscle lisse vasculaire.

20. Utilisation selon la revendication 15, dans laquelle lesdites cellules d'origine adipeuse sont élevées dans des conditions de culture qui favorisent une différenciation vers un phénotype endothélial.

21. Utilisation selon la revendication 15, dans laquelle ladite culture cellulaire est réalisée sur un matériau de support pour générer une construction bidimensionnelle ou tridimensionnelle pouvant être placée sur ou dans le coeur.

22. Utilisation selon la revendication 21, dans laquelle ledit matériau de support est résorbable in vivo.

23. Utilisation selon la revendication 1, dans laquelle les cellules progénitrices endothéliales d'origine adipeuse sont modifiées par transfert de gènes.

24. Utilisation selon la revendication 23, dans laquelle ladite modification altère l'angiogenèse.

25. Utilisation selon la revendication 23, dans laquelle ladite modification altère l'artériogenèse.

26. Utilisation selon la revendication 23, dans laquelle ladite modification altère l'apoptose.

27. Utilisation selon la revendication 26, dans laquelle lesdites cellules progénitrices endothéliales d'origine adipeuse modifiées peuvent se différencier en myocytes cardiaques à apoptose altérée.

28. Utilisation selon la revendication 23, dans laquelle ladite modification altère les propriétés de guidage desdites cellules d'origine adipeuse.
